# EUROPEAN PATENT SPECIFICATION

(11) **EP 0 929 310 B1**
(45) Date of publication and mention of the grant of the patent: **01.08.2007**
(21) Application number: 97929795.9
(22) Date of filing: 04.06.1997
(51) Int. Cl.: A61K 38/00, A61K 39/395, A61P 29/00

(54) **DIAGNOSTIC AND THERAPEUTIC METHODS RELATED TO REGULATING ENERGY MOBILIZATION WITH OB PROTEIN AND OB ANTIBODIES**
DIAGNOSTISCHE UND THERAPEUTISCHE VERFAHREN IN ZUSAMMENHANG MIT DER REGULATION DER ENERGIEMOBILISATION DURCH OB-PROTEIN UND OB-ANTIKÖRPER
PROCEDES DIAGNOSTIQUES ET THERAPEUTIQUES LIES A LA REGULATION DE MOBILISATION D'ENERGIE, PAR PROTEINE OB ET ANTICORPS OB

(30) Priority: 04.06.1996 US 18972 P
(43) Date of publication of application: 21.07.1999
(73) Proprietor: THE SCRIPPS RESEARCH INSTITUTE, La Jolla, CA 92037 (US)
(72) Inventor: FENG, Lili, San Diego, CA 92126 (US); CHEN, Sizhong, San Diego, CA 92122 (US); XIA, Yiyang, San Diego, CA 92126 (US)
(74) Representative: Fisher, Adrian John
(86) International application number: PCT/US1997/009684
(87) International publication number: WO 1997/046249

(56) References cited:
- WO-A-96/05309
- WO-A-96/35787
- SCIENCE, 28 July 1995, Vol. 269, PELLELYMOUNTER et al., "Effects of the Obese Gene Product on Body Weight Regulation in ob/ob Mice", pages 540-543. XP000602063
- SCIENCE, 28 July 1995, Vol. 269, HALAAS et al., "Weight-Reducing Effects on the Plasma Protein Encoded by the Obese Gene", pages 543-547. XP000602064
- JOURNAL OF CLINICAL INVESTIGATIONS. CLIN. INVEST., May 1996, Vol. 97, GRUNFELD et al., "Endotoxin and Cytokines Induce Expression of Leptin, the ob Gene Product, in Hamsters", pages 2152-2157. XP002965641
- JOURNAL OF EXPERIMENTAL MEDICINE, 06 January 1997, Vol. 185, Number 1, SARRAF et al., "Multiple Cytokines and Acute Inflammation Raise Mouse Leptin Levels: Potential Role in Inflammatory Anorexia", pages 171-175. XP002058097
- ARTHRITIS & RHEUMATISM, September 1996, Vol. 39, No. 9 (Supplement), ROUBENOFF et al., "TNF-alpha and Leptin in Adjuvant Arthritis (AA): Implications for Inflammatory Cachexia", page s77, Abstract No. 302. XP009006034
- PROC. NATL. ACAD. SCI. U.S.A., December 1996, Vol. 93, GAINSFORD et al., "Leptin can Induce Proliferation, Differentiation and Functional Activation of Hemopoietic Cells", pages 14564-14568. XP002921615
- CURRENT BIOLOGY, 1996, Vol. 6, No. 9, BENNETT et al., "A Role for Leptin and Its Cognate Receptor in Hematopoiesis", pages 1170-1180. XP000673008

### SEQUENCE LISTING

(1) GENERAL INFORMATION:
   (i) APPLICANT: Feng, Lili Chen, Sizhong Xia, Yiyang
   (ii) TITLE OF INVENTION: DIAGNOSTIC AND THERAPEUTIC METHODS RELATED TO REGULATING ENERGY MOBILIZATION WITH OB PROTEIN AND OB ANTIBODIES
   (iii) NUMBER OF SEQUENCES: 11
   (iv) CORRESPONDENCE ADDRESS:
      (A) ADDRESSEE: Olson & Hierl, Ltd.
      (B) STREET: 20 North Wacker Drive, 36th Floor
      (C) CITY: Chicago
      (D) STATE: IL
      (E) COUNTRY: US
      (F) ZIP: 60606
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE: 04-JUN-1997
      (C) CLASSIFICATION:
   (vii) PRIOR APPLICATION DATA:
      (A) APPLICATION NUMBER: US 60/018,972
      (B) FILING DATE: 04-JUN-1996
   (viii) ATTORNEY/AGENT INFORMATION:
      (A) NAME: Olson, Arne M
      (B) REGISTRATION NUMBER: 30,203
      (C) REFERENCE/DOCKET NUMBER: TSRI540.1PCT
   (ix) TELECOMMUNICATION INFORMATION:
      (A) TELEPHONE: 312-580-1180
      (B) TELEFAX: 312-580-1189
(2) INFORMATION FOR SEQ ID NO:1:
   (i) SEQUENCE CHARACTERISTICS:
      (A) LENGTH: 2793 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Mus musculus
   (xi) SEQUENCE DESCRIPTION: SEQ ID NO:1:
   GGATCCCTGC TCCAGCAGCT GCAAGGTGCA AGAAGAAGAA GATCCCAGGG AGGAAAATGT 60
   GCTGGAGACC CCTGTGTCGG TTCCTGTGGC TTTGGTCCTA TCTGTCTTAT GTTCAAGCAG 120
   TGCCTATCCA GAAAGTCCAG GATGACACCA AAACCCTCAT CAAGACCATT GTCACCAGGA 180
   TCAATGACAT TTCACACACG CAGTCGGTAT CCGCCAAGCA GAGGGTCACT GGCTTGGACT 240
   TCATTCCTGG GCTTCACCCC ATTCTGAGTT TGTCCAAGAT GGACCAGACT CTGGCAGTCT 300
   ATCAACAGGT CCTCACCAGC CTGCCTTCCC AAAATGTGCT GCAGATAGCC AATGACCTGG 360
   AGAATCTCCG AGACCTCCTC CATCTGCTGG CCTTCTCCAA GAGCTGCTCC CTGCCTCAGA 420
   CCAGTGGCCT GCAGAAGCCA GAGAGCCTGG ATGGCGTCCT GGAAGCCTCA CTCTACTCCA 480
   CAGAGGTGGT GGCTTTGAGC AGGCTGCAGG GCTCTCTGCA GGACATTCTT CAACAGTTGG 540
   ATGTTAGCCC TGAATGCTGA AGTTTCAAAG GCCACCAGGC TCCCAAGAAT CATGTAGAGG 600
   GAAGAAACCT TGGCTTCCAG GGGTCTTCAG GAGAAGAGAG CCATGTGCAC ACATCCATCA 660
   TTCATTTCTC TCCCTCCTGT AGACCACCCA TCCAAAGGCA TGACTCCACA ATGCTTGACT 720
   CAAGTTATCC ACACAACTTC ATGAGCACAA GGAGGGGCCA GCCTGCAGAG GGGACTCTCA 780
   CCTAGTTCTT CAGCAAGTAG AGATAAGAGC CATCCCATCC CCTCCATGTC CCACCTGCTC 840
   CGGGTACATG TTCCTCCGTG GGTACACGCT TCGCTGCGGC CCAGGAGAGG TGAGGTAGGG 900
   ATGGGTAGAG CCTTTGGGCT GTCTCAGAGT CTTTGGGAGC ACCGTGAAGG CTGCATCCAC 960
   ACACAGCTGG AAACTCCCAA GCAGCACACG ATGGAAGCAC TTATTTATTT ATTCTGCATT 1020
   CTATTTTGGA TGGATCTGAA GCAAGGCATC AGCTTTTTCA GGCTTTGGGG GTCAGCCAGG 1080
   ATGAGGAAGG CTCCTGGGGT GCTGCTTTCA ATCCTATTGA TGGGTCTGCC CGAGGCAAAC 1140
   CTAATTTTTG AGTGACTGGA AGGAAGGTTG GGATCTTCCA AACAAGAGTC TATGCAGGTA 1200
   GCGCTCAAGA TTGACCTCTG GTGACTGGTT TTGTTTCTAT TGTGACTGAC TCTATCCAAA 1260
   CACGTTTGCA GCGGCATTGC CGGGAGCATA GGCTAGGTTA TTATCAAAAG CAGATGAATT 1320
   TTGTCAAGTG TAATATGTAT CTATGTGCAC CTGAGGGTAG AGGATGTGTT AGAGGGAGGG 1380
   TGAAGGATCC GGAAGTGTTC TCTGAATTAC ATATGTGTGG TAGGCTTTTC TGAAAGGGTG 1440
   AGGCATTTTC TTACCTCTGT GGCCACATAG TGTGGCTTTG TGAAAAGGAC AAAGGAGTTG 1500
   ACTCTTTCCG GAACATTTGG AGTGTACCAG GCACCCTTGG AGGGGCTAAA GCTACAGGCC 1560
   TTTTGTTGGC ATATTGCTGA GCTCAGGGAG TGAGGGCCCC ACATTTGAGA CAGTGAGCCC 1620
   CAAGAAAAGG GTCCCTGGTG TAGATCTCCA AGGTTGTCCA GGGTTGATCT CACAATGCGT 1680
   TTCTTAAGCA GGTAGACGTT TGCATGCCAA TATGTGGTTC TCATCTGATT GGTTCATCCA 1740
   AAGTAGAACC CTGTCTCCCA CCCATTCTGT GGGGAGTTTT GTTCCAGTGG GAATGAGAAA 1800
   TCACTTAGCA GATGGTCCTG AGCCCTGGGC CAGCACTGCT GAGGAAGTGC CAGGGCCCCA 1860
   GGCCAGGCTG CCAGAATTGC CCTTCGGGCT GGAGGATGAA CAAAGGGGCT TGGGTTTTTC 1920
   CATCACCCCT GCACCCTATG TCACCATCAA ACTGGGGGGC AGATCAGTGA GAGGACACTT 1980
   GATGGAAAGC AATACACTTT AAGACTGAGC ACAGTTTCGT GCTCAGCTCT GTCTGGTGCT 2040
   GTGAGCTAGA GAAGCTCACC ACATACATAT AAAAATCAGA GGCTCATGTC CCTGTGGTTA 2100
   GACCCTACTC GCGGCGGTGT ACTCCACCAC AGCAGCACCG CACCGCTGGA AGTACAGTGC 2160
   TGTCTTCAAC AGGTGTGAAA GAACCTGAGC TGAGGGTGAC AGTGCCCAGG GGAACCCTGC 2220
   TTGCAGTCTA TTGCATTTAC ATACCGCATT TCAGGGCACA TTAGCATCCA CTCCTATGGT 2280
   AGCACACTGT TGACAATAGG ACAAGGGATA GGGGTTGACT ATCCCTTATC CAAAATGCTT 2340
   GGGACTAGAA GAGTTTTGGA TTTTAGAGTC TTTTCAGGCA TAGGTATATT TGAGTATATA 2400
   TAAAATGAGA TATCTTGGGG ATGGGGCCCA AGTATAAACA TGAAGTTCAT TTATATTTCA 2460
   TAATACCGTA TAGACACTGC TTGAAGTGTA GTTTTATACA GTGTTTTAAA TAACGTTGTA 2520
   TGCATGAAAG ACGTTTTTAC AGCATGAACC TGTCTACTCA TGCCAGCACT CAAAAACCTT 2580
   GGGGTTTTGG AGCAGTTTGG ATCTTGGGTT TTCTGTTAAG AGATGGTTAG CTTATACCTA 2640
   AAACCATAAT GGCAAACAGG CTGCAGGACC AGACTGGATC CTCAGCCCTG AAGTGTGCCC 2700
   TTCCAGCCAG GTCATACCCT GTGGAGGTGA GCGGGATCAG GTTTTGTGGT GCTAAGAGAG 2760
   GAGTTGGAGG TAGATTTTGG AGGATCTGAG GGC 2793
(2) INFORMATION FOR SEQ ID NO:2:
   (i) SEQUENCE CHARACTERISTICS:
      (A) LENGTH: 3862 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Mus musculus
   (xi) SEQUENCE DESCRIPTION: SEQ ID NO:2:
   GTCGACCCAC GCGTCCGGAG GAATCGTTCT GCAAATCCAG GTGTACACCT CTGAAGAAAG 60
   ATGATGTGTC AGAAATTCTA TGTGGTTTTG TTACACTGGG AATTTCTTTA TGTGATAGCT 120
   GCACTTAACC TGGCATATCC AATCTCTCCC TGGAAATTTA AGTTGTTTTG TGGACCACCG 180
   AACACAACCG ATGACTCCTT TCTCTCACCT GCTGGAGCCC CAAACAATGC CTCGGCTTTG 240
   AAGGGGGCTT CTGAAGCAAT TGTTGAAGCT AAATTTAATT CAAGTGGTAT CTACGTTCCT 300
   GAGTTATCCA AAACAGTCTT CCACTGTTGC TTTGGGAATG AGCAAGGTCA AAACTGCTCT 360
   GCACTCACAG ACAACACTGA AGGGAAGACA CTGGCTTCAG TAGTGAAGGC TTCAGTTTTT 420
   CGCCAGCTAG GTGTAAACTG GGACATAGAG TGCTGGATGA AAGGGGACTT GACATTATTC 480
   ATCTGTCATA TGGAGCCATT ACCTAAGAAC CCCTTCAAGA ATTATGACTC TAAGGTCCAT 540
   CTTTTATATG ATCTGCCTGA AGTCATAGAT GATTCGCCTC TGCCCCCACT GAAAGACAGC 600
   TTTCAGACTG TCCAATGCAA CTGCAGTCTT CGGGGATGTG AATGTCATGT GCCGGTACCC 660
   AGAGCCAAAC TCAACTACGC TCTTCTGATG TATTTGGAAA TCACATCTGC CGGTGTGAGT 720
   TTTCAGTCAC CTCTGATGTC ACTGCAGCCC ATGCTTGTTG TGAAACCCGA TCCACCCTTA 780
   GGTTTGCATA TGGAAGTCAC AGATGATGGT AATTTAAAGA TTTCTTGGGA CAGCCAAACA 840
   ATGGCACCAT TTCCGCTTCA ATATCAGGTG AAATATTTAG AGAATTCTAC AATTGTAAGA 900
   GAGGCTGCTG AAATTGTCTC AGCTACATCT CTGCTGGTAG ACAGTGTGCT TCCTGGATCT 960
   TCATATGAGG TCCAGGTGAG GAGCAAGAGA CTGGATGGTT CAGGAGTCTG GAGTGACTGG 1020
   AGTTCACCTC AAGTCTTTAC CACACAAGAT GTTGTGTATT TTCCACCCAA AATTCTGACT 1080
   AGTGTTGGAT CGAATGCTTC TTTTCATTGC ATCTACAAAA ACGAAAACCA GATTATCTCC 1140
   TCAAAACAGA TAGTTTGGTG GAGGAATCTA GCTGAGAAAA TCCCTGAGAT ACAGTACAGC 1200
   ATTGTGAGTG ACCGAGTTAG CAAAGTTACC TTCTCCAACC TGAAAGCCAC CAGACCTCGA 1260
   GGGAAGTTTA CCTATGACGC AGTGTACTGC TGCAATGAGC AGGCGTGCCA TCACCGCTAT 1320
   GCTGAATTAT ACGTGATCGA TGTCAATATC AATATATCAT GTGAAACTGA CGGGTACTTA 1380
   ACTAAAATGA CTTGCAGATG GTCACCCAGC ACAATCCAAT CACTAGTGGG AAGCACTGTG 1440
   CAGCTGAGGT ATCACAGGCG CAGCCTGTAT TGTCCTGATA GTCCATCTAT TCATCCTACG 1500
   TCTGAGCCCA AAAACTGCGT CTTACAGAGA GACGGCTTTT ATGAATGTGT TTTCCAGCCA 1560
   ATCTTTCTAT TATCTGGCTA TACAATGTGG ATCAGGATCA ACCATTCTTT AGGTTCACTT 1620
   GACTCGCCAC CAACGTGTGT CCTTCCTGAC TCCGTAGTAA AACCACTACC TCCATCTAAC 1680
   GTAAAAGCAG AGATTACTGT AAACACTGGA TTATTGAAAG TATCTTGGGA AAAGCCAGTC 1740
   TTTCCGGAGA ATAACCTTCA ATTCCAGATT CGATATGGCT TAAGTGGAAA AGAAATACAA 1800
   TGGAAGACAC ATGAGGTATT CGATGCAAAG TCAAAGTCTG CCAGCCTGCT GGTGTCAGAC 1860
   CTCTGTGCAG TCTATGTGGT CCAGGTTCGC TGCCGGCGGT TGGATGGACT AGGATATTGG 1920
   AGTAATTGGA GCAGTCCAGC CTATACGCTT GTCATGGATG TAAAAGTTCC TATGAGAGGG 1980
   CCTGAATTTT GGAGAAAAAT GGATGGGGAC GTTACTAAAA AGGAGAGAAA TGTCACCTTG 2040
   CTTTGGAAGC CCCTGACGAA AAATGACTCA CTGTGTAGTG TGAGGAGGTA CGTGGTGAAG 2100
   CATCGTACTG CCCACAATGG GACGTGGTCA GAAGATGTGG GAAATCGGAC CAATCTCACT 2160
   TTCCTGTGGA CAGAACCAGC GCACACTGTT ACAGTTCTGG CTGTCAATTC CCTCGGCGCT 2220
   TCCCTTGTGA ATTTTAACCT TACCTTCTCA TGGCCCATGA GTAAAGTGAG TGCTGTGGAG 2280
   TCACTCAGTG CTTATCCCCT GAGCAGCAGC TGTGTCATCC TTTCCTGGAC ACTGTCACCT 2340
   GATGATTATA GTCTGTTATA TCTGGTTATT GAATGGAAGA TCCTTAATGA AGATGATGGA 2400
   ATGAAGTGGC TTAGAATTCC CTCGAATGTT AAAAAGTTTT ATATCCACGA TAATTTTATT 2460
   CCCATCGAGA AATATCAGTT TAGTCTTTAC CCAGTATTTA TGGAAGGAGT TGGAAAACCA 2520
   AAGATAATTA ATGGTTTCAC CAAAGATGCT ATCGACAAGC AGCAGAATGA CGCAGGGCTG 2580
   TATGTCATTG TACCCATAAT TATTTCCTCT TGTGTCCTAC TGCTCGGAAC ACTGTTAATT 2640
   TCACACCAGA GAATGAAAAA GTTGTTTTGG GACGATGTTC CAAACCCCAA GAATTGTTCC 2700
   TGGGCACAAG GACTGAATTT CCAAAAGCCT GAAACATTTG AGCATCTTTT TACCAAGCAT 2760
   GCAGAATCAG TGATATTTGG TCCTCTTCTT CTGGAGCCTG AACCCATTTC AGAAGAAATC 2820
   AGTGTCGATA CAGCTTGGAA AAATAAAGAT GAGATGGTCC CAGCAGCTAT GGTCTCCCTT 2880
   CTTTTGACCA CACCAGACCC TGAAAGCAGT TCTATTTGTA TTAGTGACCA GTGTAACAGT 2940
   GCTAACTTCT CTGGGTCTCA GAGCACCCAG GTAACCTGTG AGGATGAGTG TCAGAGACAA 3000
   CCCTCAGTTA AATATGCAAC TCTGGTCAGC AACGATAAAC TAGTGGAAAC TGATGAAGAG 3060
   CAAGGGTTTA TCCATAGTCC TGTCAGCAAC TGCATCTCCA GTAATCATTC CCCACTGAGG 3120
   CAGTCTTTCT CTAGCAGCTC CTGGGAGACA GAGGCCCAGA CATTTTTCCT TTTATCAGAC 3180
   CAGCAACCCA CCATGATTTC ACCACAACTT TCATTCTCGG GGTTGGATGA GCTTTTGGAA 3240
   CTGGAGGGAA GTTTTCCTGA AGAAAATCAC AGGGAGAAGT CTGTCTGTTA TCTAGGAGTC 3300
   ACCTCCGTCA ACAGAAGAGA GAGTGGTGTG CTTTTGACTG GTGAGGCAGG AATCCTGTGC 3360
   ACATTCCCAG CCCAGTGTCT GTTCAGTGAC ATCAGGATCC TCCAGGAGAG ATGCTCACAC 3420
   TTTGTAGAAA ATAATTTGAG TTTAGGGACC TCTGGTGAGA ACTTTGTACC TTACATGCCC 3480
   CAATTTCAAA CCTGTTCCAC GCACAGTCAC AAGATAATGG AGAATAAGAT GTGTGACTTA 3540
   ACTGTGTAAT CTCATCCAAG AAGCCTCAAG GTTCCATTCC AGTAGAGCCT GTCATGTATA 3600
   ATGTGTTCTT TTATTGTTGT GGATGTGGGA GACAAGTGTC AGAATCTAGT GTGAAAATGA 3660
   TTGTTTCCAA ACTAAGTGTG TCTATTTTCT CTCAGTAATA CAATGAAACA TATGAGGAAG 3720
   CCCTCATTAA TCTAGTAATG TAGATGGACT CTTACTGAAT ATATTCCCAA GATACTTGGG 3780
   GAAGTCTCCC TAATTCTAGC TAAAAATAAA CCCAGGAATA GAACTACTAA ACACTGAATC 3840
   TGGAAAAAAA AAAAAAAAAA AG 3862
(2) INFORMATION FOR SEQ ID NO:3:
   (i) SEQUENCE CHARACTERISTICS:
      (A) LENGTH: 1974 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Mus musculus
   (xi) SEQUENCE DESCRIPTION: SEQ ID NO: 3:
   AAGTCTCCAG GGCAGAGAGG GAGTCAACTC ATTGGCGCTT GAGTCGGCAA AGAAATCAAG     60
   ATGGCCAAAG TTCCTGACTT GTTTGAAGAC CTAAAGAACT GTTACAGTGA AAACGAAGAC    120
   TACAGTTCTG CCATTGACCA TCTCTCTCTG AATCAGAAAT CCTTCTATGA TGCAAGCTAT    180
   GGCTCACTTC ATGAGACTTG CACAGATCAG TTTGTATCTC TGAGAACCTC TGAAACGTCA    240
   AAGATGTCCA ACTTCACCTT CAAGGAGAGC CGGGTGACAG TATCAGCAAC GTCAAGCAAC    300
   GGGAAGATTC TGAAGAAGAG ACGGCTGAGT TTCAGTGAGA CCTTCACTGA AGATGACCTG    360
   CAGTCCATAA CCCATGATCT GGAAGAGACC ATCCAACCCA GATCAGCACC TTACACCTAC    420
   CAGAGTGATT TGAGATACAA ACTGATGAAG CTCGTCAGGC AGAAGTTTGT CATGAATGAT    480
   TCCCTCAACC AAACTATATA TCAGGATGTG GACAAACACT ATCTCAGCAC CACTTGGTTA    540
   AATGACCTGC AACAGGAAGT AAAATTTGAC ATGTATGCCT ACTCGTCGGG AGGAGACGAC    600
   TCTAAATATC CTGTTACTCT AAAAATCTCA GATTCACAAC TGTTCGTGAG CGCTCAAGGA    660
   GAAGACCAGC CCGTGTTGCT GAAGGAGTTG CCAGAAACAC CAAAACTCAT CACAGGTAGT    720
   GAGACCGACC TCATTTTCTT CTGGAAAAGT ATCAACTCTA AGAACTACTT CACATCAGCT    780
   GCTTATCCAG AGCTGTTTAT TGCCACCAAA GAACAAAGTC GGGTGCACCT GGCACGGGGA    840
   CTGCCCTCTA TGACAGACTT CCAGATATCA TAAAAGCAGC CTTATTTCGG GAGTCTATTC    900
   ACTTGGGAAG TGCTGACAGT CTGTATGTAC CATGTACAGG AACCTTCCTC ACCCTGAGTC    960
   ACTTGCACAG CATGTGCTGA GTCTCTGTAA TTCTAAATGA ATGTTTACCC TCTTTGTAAG   1020
   AGAAGAGCAA ACCCTAGTGG AGCCACCCCG ACATATGATA CTATCTGTTA TTTTAAAGAG   1080
   TACCCTATAG TTTGCTCAGT ACTAATCATT TTAATTACTA TTCTGCATGG CATTCTTAGG   1140
   AGGATCAAAA AGACTCTACA CATATTACAG ATGGGTTAAC AAAGGGATAA AACAACTGAA   1200
   AAGCACACTC AATGCATTTG GAATATAAAT TCACAGACCA ATCTCACTGT GCACCTTCGG 1260
   CTTCAAAATG CCAGTTGAGT AGGATAAAGG TATAAGAACT TAATGCTGTC ATTTTCAAAA 1320
   GGAAGGGGAC AATAGCTACA TCTTTCCTAC CTCAGTGGGT TTTACTCCAG TGAGATCATT 1380
   TGGATGAAAT CCTCCTGTAA CAGACCTCAA GAAGGAGACA GACTGTTGAA TGTTATTTTT 1440
   AAGTTATTTT ATATATGTAT TTATAAATAT ATTTATGATA ATTATATTAT TTATGGAACA 1500
   TCCTTAAATC CTCTGAGCTT GACAGGCATC CTCACAGCAG GATTTTCTAG GTGGTCAGTT 1560
   AGATATAGTT TCCTCTAGAG CACCATGCTA CAGACTTTAC ACTTTTTCCA CAGCCACGAA 1620
   GCTCTCTGTA CATTCCTGTA CTTGGGAGCC CTTTCATCAT GATCTTAATC TGTACTGTTT 1680
   ACTTTGTTCA TCTAAAATGA TAATTGAGTC AGTCTTTTTC CCTCCCATCC TTAAAGCTGT 1740
   CTGGGTATTC TTACATCATT CAGTCTCACC TGTAACTAAC ACCAACCATC TAAAGATGGA 1800
   AAGAGCTTAA CTGTGACAAC CACATCACTG TTACCTGAAG TTTCTTTTCT AGAATGTAAT 1860
   CAGTGTTTCC CCTGGATTCC AATTTTTTTT TCAAACCACA GTATCATGTA ACTATCAACA 1920
   ATAACAATCA ACTCATTATT ATTAATCATA ATTAAATAAA ACAAGTTTGA GCTG 1974
(2) INFORMATION FOR SEQ ID NO:4:
   (i) SEQUENCE CHARACTERISTICS:
      (A) LENGTH: 1339 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Mus musculus
   (xi) SEQUENCE DESCRIPTION: SEQ ID NO:4:
   TGCAGGGTTC GAGGCCTAAT AGGCTCATCT GGGATCCTCT CCAGCCAAGC TTCCTTGTGC 60
   AAGTGTCTGA AGCAGCTATG GCAACTGTTC CTGAACTCAA CTGTGAAATG CCACCTTTTG 120
   ACAGTGATGA GAATGACCTG TTCTTTGAAG TTGACGGACC CCAAAAGATG AAGGGCTGCT 180
   TCCAAACCTT TGACCTGGGC TGTCCAGATG AGAGCATCCA GCTTCAAATC TCACAGCAGC 240
   ACATCAACAA GAGCTTCAGG CAGGCAGTAT CACTCATTGT GGCTGTGGAG AAGCTGTGGC 300
   AGCTACCTGT GTCTTTCCCG TGGACCTTCC AGGATGAGGA CATGAGCACC TTCTTTTCCT 360
   TCATCTTTGA AGAAGAGCCC ATCCTCTGTG ACTCATGGGA TGATGATGAT AACCTGCTGG 420
   TGTGTGACGT TCCCATTAGA CAGCTGCACT ACAGGCTCCG AGATGAACAA CAAAAAAGCC 480
   TCGTGCTGTC GGACCCATAT GAGCTGAAAG CTCTCCACCT CAATGGACAG AATATCAACC    540
   AACAAGTGAT ATTCTCCATG AGCTTTGTAC AAGGAGAACC AAGCAACGAC AAAATACCTG    600
   TGGCCTTGGG CCTCAAAGGA AAGAATCTAT ACCTGTCCTG TGTAATGAAA GACGGCACAC    660
   CCACCCTGCA GCTGGAGAGT GTGGATCCCA AGCAATACCC AAAGAAGAAG ATGGAAAAGC    720
   GGTTTGTCTT CAACAAGATA GAAGTCAAGA GCAAAGTGGA GTTTGAGTCT GCAGAGTTCC    780
   CCAACTGGTA CATCAGCACC TCACAAGCAG AGCACAAGCC TGTCTTCCTG GGAAACAACA    840
   GTGGTCAGGA CATAATTGAC TTCACCATGG AATCTGTGTC TTCCTAAAGT ATGGGCTGGA    900
   CTGTTTCTAA TGCCTTCCCC AGGGCATGTG AAGGAGCTCC CTTGTCATGA ATGAGCAGAC    960
   AGCTCAATCT CTAGGACACT CCTTAGTCCT CGGCCAAGAC AGGTCGCTCA GGGTCACAAG   1020
   AAACCATGGC ACATTCTGTT CAAAGAGAGC CTGTGTTTCC TCCTTGCCTC TGATGGGCAA   1080
   CCACTTACCT ATTTATTTAT GTATTTATTG ATTGGTTGAT CTATTTAAGT TGATTCAAGG   1140
   GGACATTAGG CAGCACTCTC TAGAACAGAA CCTAGCTGTC AACGTGTGGG GGATGAATTG   1200
   GTCATAGCCT TGCACTTGAG GTCTTTCATT GAAGCTGAGA ATAAATAGGT TCCTATAATA   1260
   TGGATGAGAA TTTTTATGAA TGAAGCATTA GCACATTGCT TTGATGAGTA TGAAATAAAT   1320
   TTCATTAAAC AAACAAACA                                                 1339
(2) INFORMATION FOR SEQ ID NO:5:
   (i) SEQUENCE CHARACTERISTICS:
      (A) LENGTH: 1629 base pairs
      (B) TYPE : nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Mus musculus
   (xi) SEQUENCE DESCRIPTION: SEQ ID NO:5:
   GCTGAGGGAC TAGCCAGGAG GGAGAACAGA AACTCCAGAA CATCCTGGAA ATAGCTCCCA     60
   GAAAAGCAAG CAGCCAACCA GGCAGGTTCT GTCCCTTTCA CTCACTGGCC CAAGGCGCCA    120
   CATCTCCCTC CAGAAAAGAC ACCATGAGCA CAGAAAGCAT GATCCGCGAC GTGGAACTGG    180
   CAGAAGAGGC ACTCCCCCAA AAGATGGGGG GCTTCCAGAA CTCCAGGCGG TGCCTATGTC    240
   TCAGCCTCTT CTCATTCCTG CTTGTGGCAG GGGCCACCAC GCTCTTCTGT CTACTGAACT    300
   TCGGGGTGAT CGGTCCCCAA AGGGATGAGA AGTTCCCAAA TGGCCTCCCT CTCATCAGTT    360
   CTATGGCCCA GACCCTCACA CTCAGATCAT CTTCTCAAAA TTCGAGTGAC AAGCCTGTAG     420
   CCCACGTCGT AGCAAACCAC CAAGTGGAGG AGCAGCTGGA GTGGCTGAGC CAGCGCGCCA     480
   ACGCCCTCCT GGCCAACGGC ATGGATCTCA AAGACAACCA ACTAGTGGTG CCAGCCGATG     540
   GGTTGTACCT TGTCTACTCC CAGGTTCTCT TCAAGGGACA AGGCTGCCCC GACTACGTGC     600
   TCCTCACCCA CACCGTCAGC CGATTTGCTA TCTCATACCA GGAGAAAGTC AACCTCCTCT     660
   CTGCCGTCAA GAGCCCCTGC CCCAAGGACA CCCCTGAGGG GGCTGAGCTC AAACCCTGGT     720
   ATGAGCCCAT ATACCTGGGA GGAGTCTTCC AGCTGGAGAA GGGGGACCAA CTCAGCGCTG     780
   AGGTCAATCT GCCCAAGTAC TTAGACTTTG CGGAGTCCGG GCAGGTCTAC TTTGGAGTCA     840
   TTGCTCTGTG AAGGGAATGG GTGTTCATCC ATTCTCTACC CAGCCCCCAC TCTGACCCCT     900
   TTACTCTGAC CCCTTTATTG TCTACTCCTC AGAGCCCCCA GTCTGTGTCC TTCTAACTTA     960
   GAAAGGGGAT TATGGCTCAG AGTCCAACTC TGTGCTCAGA GCTTTCAACA ACTACTCAGA    1020
   AACACAAGAT GCTGGGACAG TGACCTGGAC TGTGGGCCTC TCATGCACCA CCACCCACGG    1080
   AATCGAGAAA GAGCTATCAA TCTGGAATTC ACTGGAGCCT CGAATGTCCA TTCCTGAGTT    1140
   CTGCAAAGGG AGAGTGGTCA GGTTGCCTCT GTCTCAGAAT GAGGCTGGAT AAGATCTCAG    1200
   GCCTTCCTAC CTTCAGACCT TTCCAGACTC TTCCCTGAGG TGCAATGCAC AGCCTTCCTC    1260
   ACAGAGCCAG CCCCCCTCTA TTTATATTTG CACTTATTAT TTATTATTTA TTTATTATTT    1320
   ATTTATTTGC TTATGAATGT ATTTATTTGG AAGGCCGGGG TGTCCTGGAG GACCCAGTGT    1380
   GGGAAGCTGT CTTCAGACAG ACATGTTTTC TGTGAAAACG GAGCTGAGCT GTCCCCACCT    1440
   GGCCTCTCTA CCTTGTTGCC TCCTCTTTTG CTTATGTTTA AAACAAAATA TTTATCTAAC    1500
   CCAATTGTCT TAATAACGCT GATTTGGTGA CCAGGCTGTC GCTACATCAC TGAACCTCTG    1560
   CTCCCCACGG GAGCCGTGAC TGTAATTGCC CTACGGGTCA TTGAGAGAAA TAAAGATCGC    1620
   TTGGAAAAG                                                           1629
(2) INFORMATION FOR SEQ ID NO:6:
   (i) SEQUENCE CHARACTERISTICS:
      (A) LENGTH: 4110 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Mus musculus
   (xi) SEQUENCE DESCRIPTION: SEQ ID NO:6:
   GAGACTCTGG CCCCACGGGA CACAGTGTCA CTGGTTTGAA ACTTCTCAGC CACCTTGGTG    60
   AAGGGACTGA GCTGTTAGAG ACACTTCTGA GGCTCCTCAC GCTTGGGTCT TGTTCACTCC   120
   ACGGAGTAGC CTAGTCAACT GCAAGAGAAC GGAGAACGTT GGATTTGGAG CAGAAGTGCA   180
   AAGTCTCAGA CATGGCTTGC CCCTGGAAGT TTCTCTTCAA AGTCAAATCC TACCAAAGTG   240
   ACCTGAAAGA GGAAAAGGAC ATTAACAACA ACGTGAAGAA AACCCCTTGT GCTGTTCTCA   300
   GCCCAACAAT ACAAGATGAC CCTAAGAGTC ACCAAAATGG CTCCCCGCAG CTCCTCACTG   360
   GGACAGCACA GAATGTTCCA GAATCCCTGG ACAAGCTGCA TGTGACATCG ACCCGTCCAC   420
   AGTATGTGAG GATCAAAAAC TGGGGCAGTG GAGAGATTTT GCATGACACT CTTCACCACA   480
   AGGCCACATC GGATTTCACT TGCAAGTCCA AGTCTTGCTT GGGGTCCATC ATGAACCCCA   540
   AGAGTTTGAC CAGAGGACCC AGAGACAAGC CTACCCCTCT GGAGGAGCTC CTGCCTCATG   600
   CCATTGAGTT CATCAACCAG TATTATGGCT CCTTTAAAGA GGCAAAAATA GAGGAACATC   660
   TGGCCAGGCT GGAAGCTGTA ACAAAGGAAA TAGAAACAAC AGGAACCTAC CAGCTCACTC   720
   TGGATGAGCT CATCTTTGCC ACCAAGATGG CCTGGAGGAA TGTCCCTCGC TGCATCGGCA   780
   GGATCCAGTG GTCCAACCTG CAGGTCTTTG ACGCTCGGAA CTGTAGCACA GCACAGGAAA   840
   TGTTTCAGCA CATCTGCAGA CACATACTTT ATGCCACCAA CAATGGCAAC ATCAGGTCGG   900
   CCATCACTGT GTTCCCCCAG CGGAGTGACG GCAAACATGA CTTCAGGCTC TGGAATTCAC   960
   AGCTCATCCG GTACGCTGGC TACCAGATGC CCGATGGCAC CATCAGAGGG GATGCTGCCA  1020
   CCTTGGAGTT CACCCAGTTG TGCATCGACC TAGGCTGGAA GCCCCGCTAT GGCCGCTTTG  1080
   ATGTGCTGCC TCTGGTCTTG CAAGCTGATG GTCAAGATCC AGAGGTCTTT GAAATCCCTC  1140
   CTGATCTTGT GTTGGAGGTG ACCATGGAGC ATCCCAAGTA CGAGTGGTTC CAGGAGCTCG  1200
   GGTTGAAGTG GTATGCACTG CCTGCCGTGG CCAACATGCT ACTGGAGGTG GGTGGCCTCG  1260
   AATTCCCAGC CTGCCCCTTC AATGGTTGGT ACATGGGCAC CGAGATTGGA GTTCGAGACT  1320
   TCTGTGACAC ACAGCGCTAC AACATCCTGG AGGAAGTGGG CCGAAGGATG GGCCTGGAGA  1380
   CCCACACACT GGCCTCCCTC TGGAAAGACC GGGCTGTCAC GGAGATCAAT GTGGCTGTGC  1440
   TCCATAGTTT CCAGAAGCAG AATGTGACCA TCATGGACCA CCACACAGCC TCAGAGTCCT  1500
   TCATGAAGCA CATGCAGAAT GAGTACCGGG CCCGTGGAGG CTGCCCGGCA GACTGGATTT  1560
   GGCTGGTCCC TCCAGTGTCT GGGAGCATCA CCCCTGTGTT CCACCAGGAG ATGTTGAACT  1620
   ATGTCCTATC TCCATTCTAC TACTACCAGA TCGAGCCCTG GAAGACCCAC ATCTGGCAGA  1680
   ATGAGAAGCT GAGGCCCAGG AGGAGAGAGA TCCGATTTAG AGTCTTGGTG AAAGTGGTGT  1740
   TCTTTGCTTC CATGCTAATG CGAAAGGTCA TGGCTTCACG GGTCAGAGCC ACAGTCCTCT  1800
   TTGCTACTGA GACAGGGAAG TCTGAAGCAC TAGCCAGGGA CCTGGCCACC TTGTTCAGCT 1860
   ACGCCTTCAA CACCAAGGTT GTCTGCATGG ACCAGTATAA GGCAAGCACC TTGGAAGAGG 1920
   AGCAACTACT GCTGGTGGTG ACAAGCACAT TTGGGAATGG AGACTGTCCC AGCAATGGGC 1980
   AGACTCTGAA GAAATCTCTG TTCATGCTTA GAGAACTCAA CCACACCTTC AGGTATGCTG 2040
   TGTTTGGCCT TGGCTCCAGC ATGTACCCTC AGTTCTGCGC CTTTGCTCAT GACATCGACC 2100
   AGAAGCTGTC CCACCTGGGA GCCTCTCAGC TTGCCCCAAC AGGAGAAGGG GACGAACTCA 2160
   GTGGGCAGGA GGATGCCTTC CGCAGCTGGG CTGTACAAAC CTTCCGGGCA GCCTGTGAGA 2220
   CCTTTGATGT CCGAAGCAAA CATCACATTC AGATCCCGAA ACGCTTCACT TCCAATGCAA 2280
   CATGGGAGCC ACAGCAATAT AGGCTCATCC AGAGCCCGGA GCCTTTAGAC CTCAACAGAG 2340
   CCCTCAGCAG CATCCATGCA AAGAACGTGT TTACCATGAG GCTGAAATCC CAGCAGAATC 2400
   TGCAGAGTGA AAAGTCCAGC CGCACCACCC TCCTCGTTCA GCTCACCTTC GAGGGCAGCC 2460
   GAGGGCCCAG CTACCTGCCT GGGGAACACC TGGGGATCTT CCCAGGCAAC CAGACCGCCC 2520
   TGGTGCAGGG AATCTTGGAG CGAGTTGTGG ATTGTCCTAC ACCACACCAA ACTGTGTGCC 2580
   TGGAGGTTCT GGATGAGAGC GGCAGCTACT GGGTCAAAGA CAAGAGGCTG CCCCCCTGCT 2640
   CACTCAGCCA AGCCCTCACC TACTTCCTGG ACATTACGAC CCCTCCCACC CAGCTGCAGC 2700
   TCCACAAGCT GGCTCGCTTT GGCACGGACG AGACGGATAG GCAGAGATTG GAGGCCTTGT 2760
   GTCAGCCCTC AGAGTACAAT GACTGGAAGT TCAGCAACAA CCCCACGTTC CTGGAGGTGC 2820
   TTGAAGAGTT CCCTTCCTTG CATGTGCCCG CTGCCTTCCT GCTGTCGCAG CTCCCTATCT 2880
   TGAAGCCCCG CTACTACTCC ATCAGCTCCT CCCAGGACCA CACCCCCTCG GAGGTTCACC 2940
   TCACTGTGGC CGTGGTCACC TACCGCACCC GAGATGGTCA GGGTCCCCTG CACCATGGTG 3000
   TCTGCAGCAC TTGGATCAGG AACCTGAAGC CCCAGGACCC AGTGCCCTGC TTTGTGCGAA 3060
   GTGTCAGTGG CTTCCAGCTC CCTGAGGACC CCTCCCAGCC TTGCATCCTC ATTGGGCCTG 3120
   GTACGGGCAT TGCTCCCTTC CGAAGTTTCT GGCAGCAGCG GCTCCATGAC TCCCAGCACA 3180
   AAGGGCTCAA AGGAGGCCGC ATGAGCTTGG TGTTTGGGTG CCGGCACCCG GAGGAGGACC 3240
   ACCTCTATCA GGAAGAAATG CAGGAGATGG TCCGCAAGAG AGTGCTGTTC CAGGTGCACA 3300
   CAGGCTACTC CCGGCTGCCC GGCAAACCCA AGGTCTACGT TCAGGACATC CTGCAAAAGC 3360
   AGCTGGCCAA TGAGGTACTC AGCGTTCTCC ACGGGGAGCA GGGCCACCTC TACATTTGCG 3420
   GAGATGTGCG CATGGCTCGG GATGTGGCTA CCACATTGAA GAAGCTGGTG GCCACCAAGC 3480
   TGAACTTGAG CGAGGAGCAG GTGGAAGACT ATTTCTTCCA GCTCAAGAGC CAGAAACGTT 3540
   ATCATGAAGA TATCTTCGGT GCAGTCTTTT CCTATGGGGC AAAAAAGGGC AGCGCCTTGG 3600
   AGGAGCCCAA AGCCACGAGG CTCTGACAGC CCAGAGTTCC AGCTTCTGGC ACTGAGTAAA 3660
   GATAATGGTG AGGGGCTTGG GGAGACAGCG AAATGCAATC CCCCCCAAGC CCCTCATGTC 3720
   ATTCCCCCCT CCTCCACCCT ACCAAGTAGT ATTGTATTAT TGTGGACTAC TAAATCTCTC 3780
   TCCTCTCCTC CCTCCCCTCT CTCCCTTTCC TCCCTTCTTC TCCACTCCCC AGCTCCCTCC 3840
   TTCTCCTTCT CCTCCTTTGC CTCTCACTCT TCCTTGGAGC TGAGAGCAGA GAAAAACTCA 3900
   ACCTCCTGAC TGAAGCACTT TGGGTGACCA CCAGGAGGCA CCATGCCGCC GCTCTAATAC 3960
   TTAGCTGCAC TATGTACAGA TATTTATACT TCATATTTAA GAAAACAGAT ACTTTTGTCT 4020
   ACTCCCAATG ATGGCTTGGG CCTTTCCTGT ATAATTCCTT GATGAAAAAT ATTTATATAA 4080
   AATACATTTT ATTTTAATCA AAAAAAAAAA 4110
(2) INFORMATION FOR SEQ ID NO:7:
   (i) SEQUENCE CHARACTERISTICS:
      (A) LENGTH: 465 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Rattus norvegicus
   (xi) SEQUENCE DESCRIPTION: SEQ ID NO:7:
   GGCATCATGG CTGCCCTTCG GCCTCTGGTG AAGCCCAAGA TCGTCAAAAA GAGGACCAAG 60
   AAGTTCATCA GGCACCAGTC GGACCGATAT GTGAAAATTA AGCGAAACTG GCGGAAACCC 120
   AGAGGCATCG ACAACAGGGT GCGGAGAAGA TTCAAGGGCC AGATCCTGAT GCCCAACATT 180
   GGTTACGGGA GTAACAAGAA AACCAAGCAC ATGCTGCCTA GCGGCTTCCG GAAGTTTCTG 240
   GTCCACAATG TCAAGGAGCT GGAAGTGCTG CTGATGTGCA ACAAATCTTA CTGTGCTGAG 300
   ATTGCTCACA ATGTGTCCTC TAAGAACCGA AAAGCCATCG TAGAAAGAGC AGCACAGCTG 360
   GCCATCAGAG TCACCAATCC CAACGCCAGG CTACGCAGCG AAGAGAATGA ATAGATGGCT 420
   TGTGTGCCTG TTTTGTGTTC AAATAAAACC ACAAAAACTG CCAAA 465
(2) INFORMATION FOR SEQ ID NO:8:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Mus musculus
   (xi) SEQUENCE DESCRIPTION: SEQ ID NO:8:
      GCTATCGACA AGCAGCAGAA T 21
(2) INFORMATION FOR SEQ ID NO:9:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Mus musculus
   (xi) SEQUENCE DESCRIPTION: SEQ ID NO:9:
      TGAACACAAC AACATAAAGC CC 22
(2) INFORMATION FOR SEQ ID NO:10:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Mus musculus
   (xi) SEQUENCE DESCRIPTION: SEQ ID HO:10:
      TGTTATATCT GGTTATTATT GAATGG 26
(2) INFORMATION FOR SEQ ID NO:11:
   (i) SEQUENCE CHARACTERISTICS:
      (A) LENGTH: 27 base pairs
      (B) TYPE: nucleic acid
      (C) STRANDEDNESS : single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Mus musculus
   (xi) SEQUENCE DESCRIPTION: SEQ ID NO:11:
      CATTAAATGA TTTATTATCA GAATTGC 27

## Description

### Reference to Related Application

This application claims the benefit of U.S. Provisional Application S.N. 60/018,972, filed June 4, 1996.

### Governmental Rights

This invention was made with governmental support from the United States Government, National Institutes of Health, Grant DK20043; the United States Government has certain rights in the invention.

### Background of the Invention

The *obese* gene in human, rat and mouse encodes a protein hormone having an open reading frame 167 amino acid residues in length, called leptin, also known as OB protein or the *ob* gene product. Removal of the signal sequence yields a mature secreted 16 kilodalton protein that is 146 amino acid residues in length.

OB protein is produced primarily by adipocytes of white adipose tissue (WAT). OB protein is secreted directly into the extracellular space and travels through the blood stream. OB protein affects the cells of its target organs by binding to the OB receptor protein, OB-R, that is found on the extracellular surface of the plasma membrane of target cells. Binding of OB protein to OB-R activates the intracellular second messenger cascade of the JAK-STAT system, which is characteristic of activation of cytokine type I receptors.

OB protein is produced in adipocytes in proportion to the mass of stored fat, thereby providing a hormone signal for a lipostatic feedback circuit, which is mediated by the OB receptor. While OB proteins of different species show a close similarity in their sequences, the sequences of OB proteins are not closely similar to other types of proteins. For example, the human ob gene sequence and its mouse homologue (85 % sequence identity) have been reported to have no sequence similarity to other proteins of known structure (DiFrancesco, V., et al., Protein Topology Recognition from Secondary Structure Sequences: Application of the Hidden Markov Models to Alpha Class Proteins, J. Mol. Biol. 267: 446-463 (1997) at page 457).

Although OB protein is composed of a single peptide chain, an intrachain disulfide bond between cysteine 96 and cysteine 146 is required both to stabilize the conformation of the molecule and to confer in vivo biological activity (Rock, F.L., et al., The Leptin Haemopoietic Cytokine Fold is Stabilized by an Intrachain Disulfide Bond, Horm. Metab. Res. 28: 649-652 (1996)). It is believed that the special geometry of the A and D major helices must be maintained in order to dock to a conserved receptor trough in the receptor molecule, a requirement that produces structural similarity between OB proteins and cytokines in the face of negligible sequence conservation (Id. at 651.).

One accepted and successful animal model of human obesity is the genetically obese mouse bearing the recessive *obese* mutation *(ob*/*ob).* The mouse model reproduces not only the human obesity condition, but also develops non-insulin dependent diabetes mellitus (NIDDM, also known as type II diabetes mellitus). Homologous *obese* genes have been described in mouse, rat and human.

The mouse is also a widely accepted and successful model of sepsis, septic shock and systemic inflammatory response syndrome (SIRS), a term which describes the clinical syndrome of sepsis without regard to its cause. Simple models, involving a large bolus dose of lipopolysaccharide (LPS) administered to mice and using mortality as the primary outcome variable, are well suited for preliminary pharmacological studies of new drugs or other therapeutic agents (Fink, M.P. & Heard, S.O., Laboratory Models of Sepsis and Septic Shock, J. Surg. Res. 49: 186-196, 1990, at 188-189).

Both the *ob* gene (Zhang, Y., et a ; ., Positional cloning of the mouse obese gene and its human homologue, Nature 372: 425-432 (1994); accession No. U18812, SEQ ID NO. 1) and its receptor (Tartaglia, L.A., et al., Identification and expression cloning of a Leptin Receptor, Cell 83: 1263-1271 (1995); Chen, H., et al., Evidence that the Diabetes Gene Encodes the Leptin Receptor: Identification of a Mutation in the Leptin Receptor Gene in db/db Mice Cell 84: 491-495 (1996); accession No. U46135, SEQ ID NO. 2) have been cloned. Shorter versions of the OB receptor, termed the OB-Ra, OB-Rc and OB-Re forms, are produced by alternative splicing of the OB-R mRNA (Lee, G. H., et al. Nature 379: 632-635 (1996)). The full length OB receptor is called the OB-Rb form.

Neural activity in specific regions of the central nervous system (CNS), such as the hypothalamus, controls processes related to food intake and energy expenditure. The cloning of the OB protein gene and the OB receptor gene and the localization of OB receptor expression in the hypothalamus has provided supporting evidence for this view as well as suggesting possible mechanisms for relating food intake to stored fat reserves. The OB protein is produced by adipocytes in proportion to the mass of stored fat and, hence, it acts as the signal to a lipostat control circuit. This lipostat signal is transduced at the target cells by the OB receptor, OB-R, in the CNS, resulting changes in neural activity that regulate both food intake and metabolic rate.

Metabolic derangement is an important characteristic of the host response to critical illness called the acute phase response that characterizes conditions such as sepsis and septic shock (Kushner, I. Ann. N.Y. Acad. Sci. 389: 39-48 (1982)). Hypothermia is a metabolic response that may be pertinent clinical prognostic factor in systemic inflammatory response syndrome in humans (Brivet, F., et al. Crit. Care Med. 22: 533-534 (1994)).

There is a need for at least one disease marker for systemic inflammatory response syndrome (SIRS) and related conditions. Hereinafter, the term SIRS is used to denote sepsis, septic shock, sepsis syndrome, and related conditions. Disease markers have numerous functions. In this case, a marker for SIRS would be useful for predicting the development of SIRS, identifying patients with SIRS, predicting outcome, aiding timing and targeting of therapeutic interventions, and determining the pathogenesis of SIRS in patients (Parsons, P.E. & Moss, M. Early Detection and Markers of Sepsis, Clinics in Chest Medicine 17:199-212 (1996)).

### Summary of the Invention

The present invention relates to the use of an OB-R agonist ligand in the manufacture of medicaments, as claimed in claim 1.

Suitable OB-R agonist ligands include recombinant OB protein, peptide conformational analogs of human OB protein comprising conservative substitutions of amino acid residues and OB-related peptides. A preferred OB-R agonist ligand is recombinant human OB protein.

Antibodies to OB protein are useful as an assay kit and method for detecting the level of OB protein in a patient. The level of OB protein in a patient is a disease marker that is useful for predicting the development of a condition, identifying patients with the condition, predicting outcome of the condition, aiding timing and targeting of therapeutic interventions, and determining the pathogenesis of the condition in patients. Conditions in which the level of OB protein is a useful marker are SIRS and related conditions such as sepsis and septic shock.

### Brief Summary of the Drawings

In the drawings:

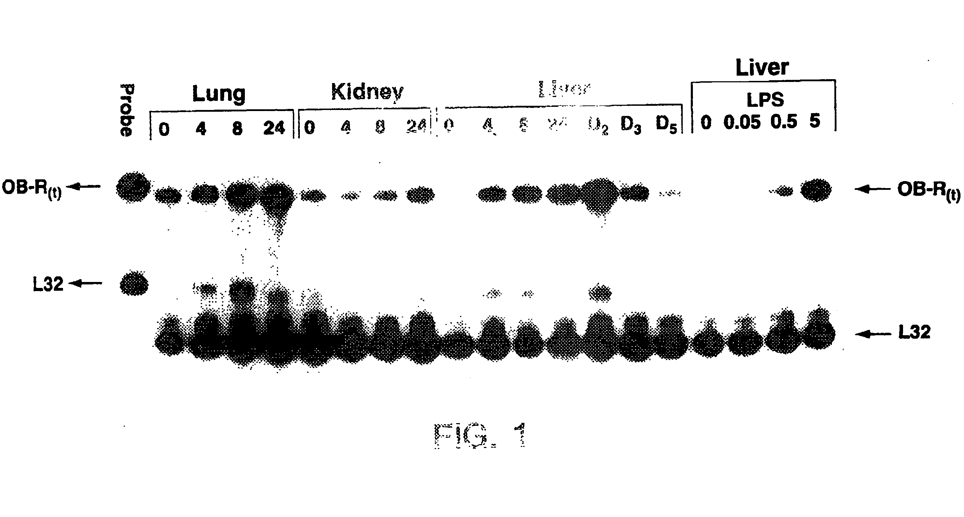

Figure 1 is a representation of an autoradiogram showing the results of a ribonuclease (RNase) protection assay showing expression of total OB receptor (OB-R₍ₜ₎) in lung, kidney and liver at 0, 4, 8 or 24 hours, or 2 (D₂), 3 (D₃) or 5 (D₅) days after intravenous injection of 5 µg per gram of body weight as well as a dose-response study showing the relative effects on the liver the of injection of 0.05, 0.5 or 5 µg of LPS per gram of body weight;

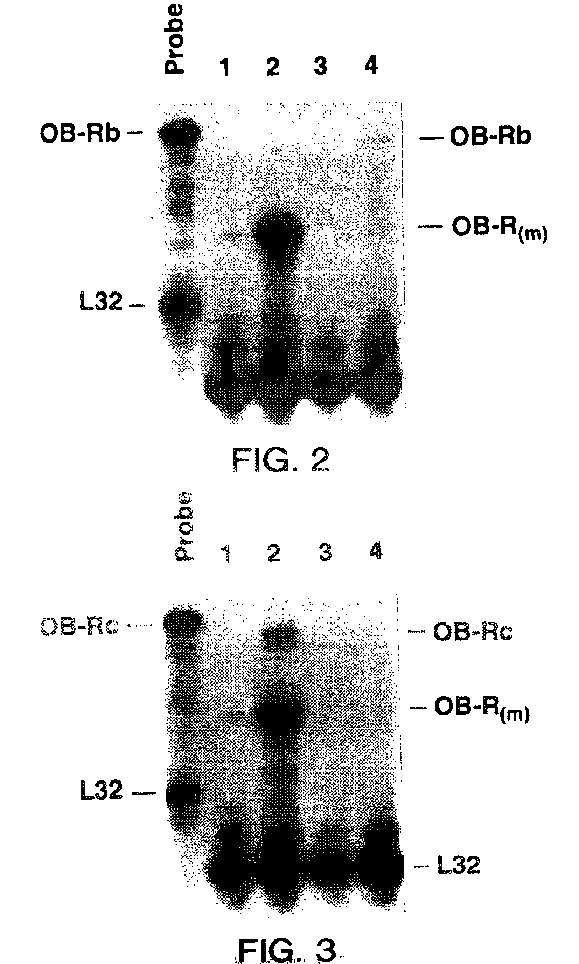

Figure 2 is a representation of an autoradiogram showing of the results of a RNase protection assay showing expression of OB-Rb in normal liver (lane 1), and the LPS-treated liver at 24 hours (lane 2), and in the hypothalamus of normal control (lane 3) and *ob*/*ob* mice (lane 4), compared to OB-R₍ₘ₎) which represents the mixture of OB-R forms partially protected by the designated nucleotide probes;
Figure 3 is a representation of an autoradiogram showing the results of a RNase protection assay showing expression of OB-Rc in normal liver (lane 1), and the LPS-treated liver at 24 hours (lane 2), and in the hypothalamus of normal control (lane 3) and *ob*/*ob* mice (lane 4), compared to DB-R₍ₘ₎) which represents the mixture of OB-R forms partially protected by the designated nucleotide probes;

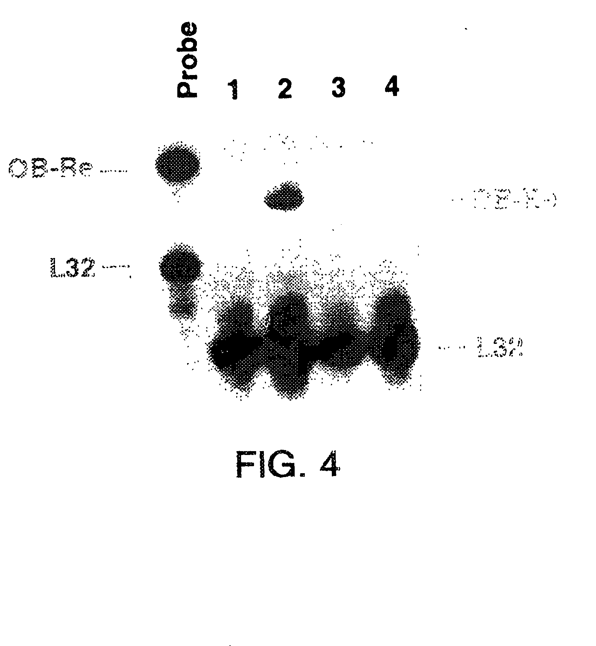

Figure 4 is a representation of an autoradiogram showing the results of a RNase protection assay showing expression of OB-Re in normal liver (lane 1), and the LPS-treated liver at 24 hours (lane 2), and in the hypothalamus of normal control (lane 3) and *ob*/*ob* mice (lane 4), compared to OB-R₍ₘ₎) which represents the mixture of OB-R forms partially protected by the designated nucleotide probes;

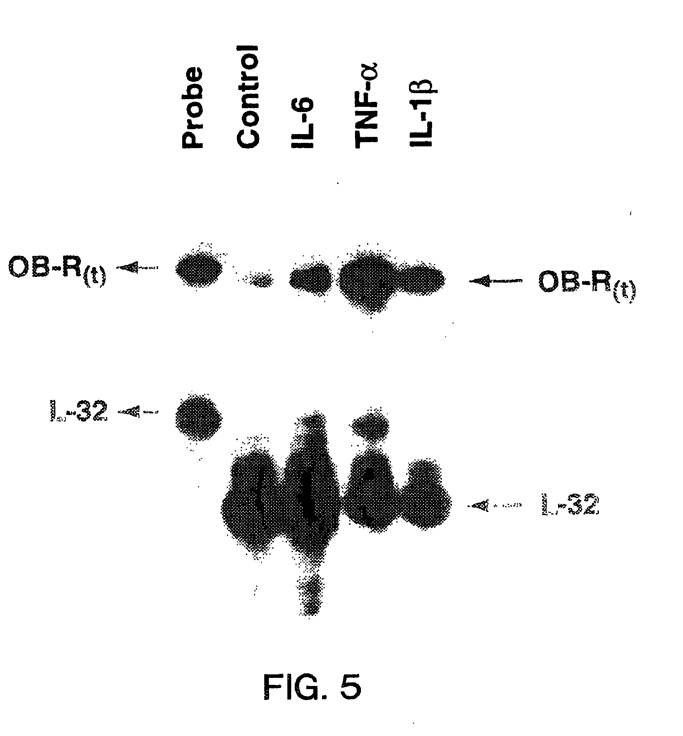

Figure 5 is a representation of an autoradiogram showing the results of a RNase protection assay showing OB-R expression in mouse liver 24 hours after IL-6 (2.5 µg per mouse), TNF-α (10 µg per mouse) and IL-1β (5 µg per mouse) injection;

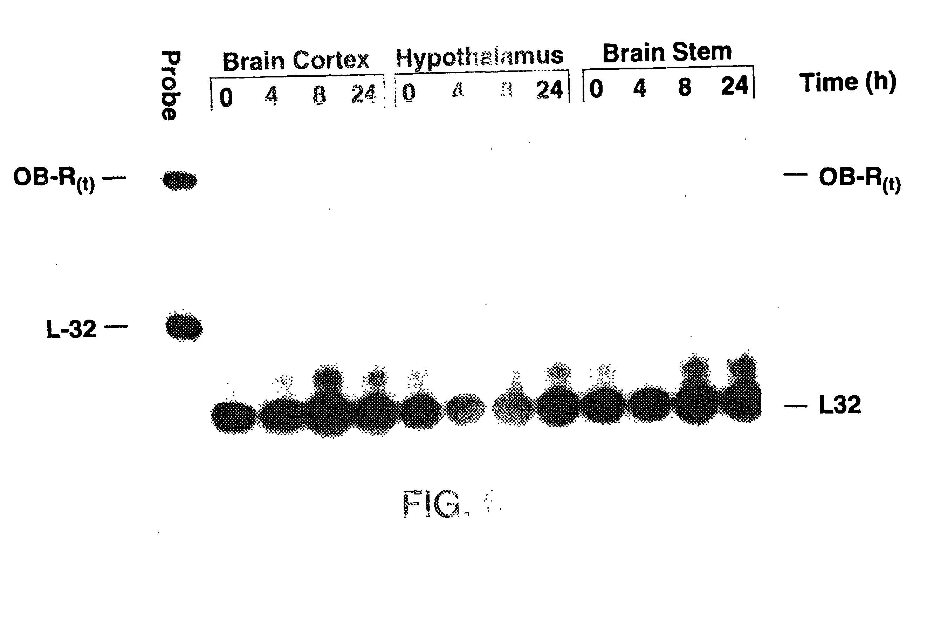

Figure 6 is a representation of an autoradiogram showing the results of a RNase protection assay showing OB-R mRNA levels in brain cortex, hypothalamus, and brain stem at various times after LPS injection (5 µg per gram of body weight);

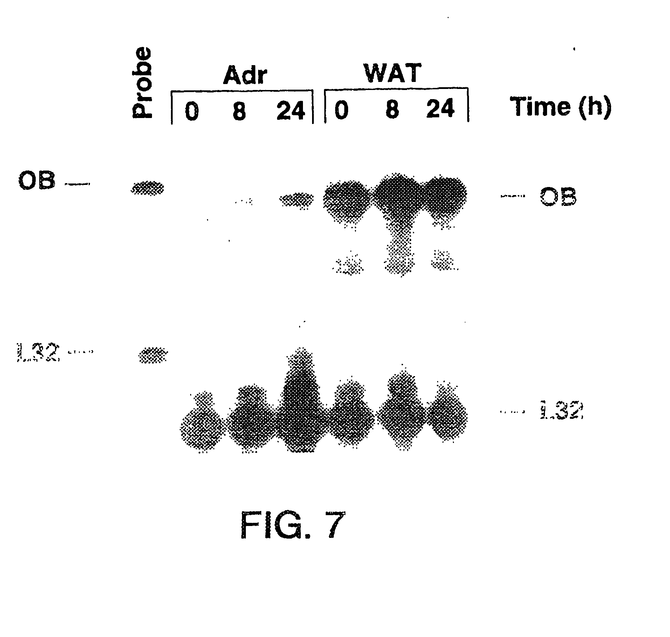

Figure 7 is a representation of an autoradiogram showing the results of a RNase protection assay showing OB mRNA expression in the adrenal gland (Adr) and white adipose tissue (WAT) at various times after LPS injection (5 µg per gram of body weight);

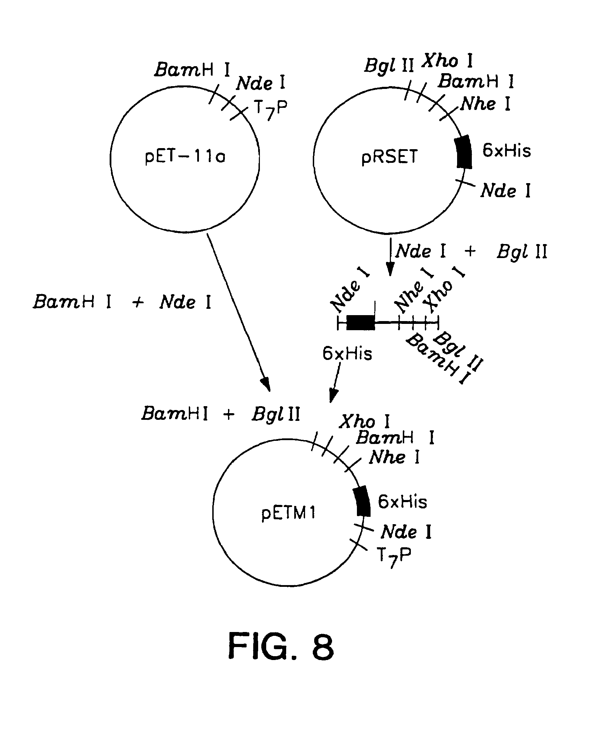

Figure 8 is a graphical representation of the construction scheme for the vector pETM1 from a commercially available vector;

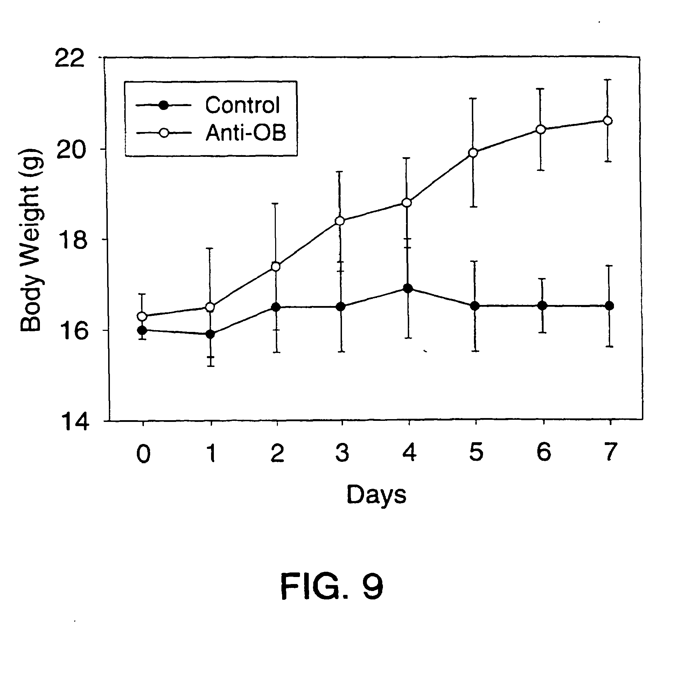

Figure 9 is a graphical representation of the weight gain induced by anti-OB antiserum injection in C57BL/6 mice which were given daily injections of anti-OB antiserum (antiOB) or preimmune rabbit sera (control), and whose body weight was measured 12 hours later, where data are expressed as mean ± standard error of the mean (S.E.M), N=8;

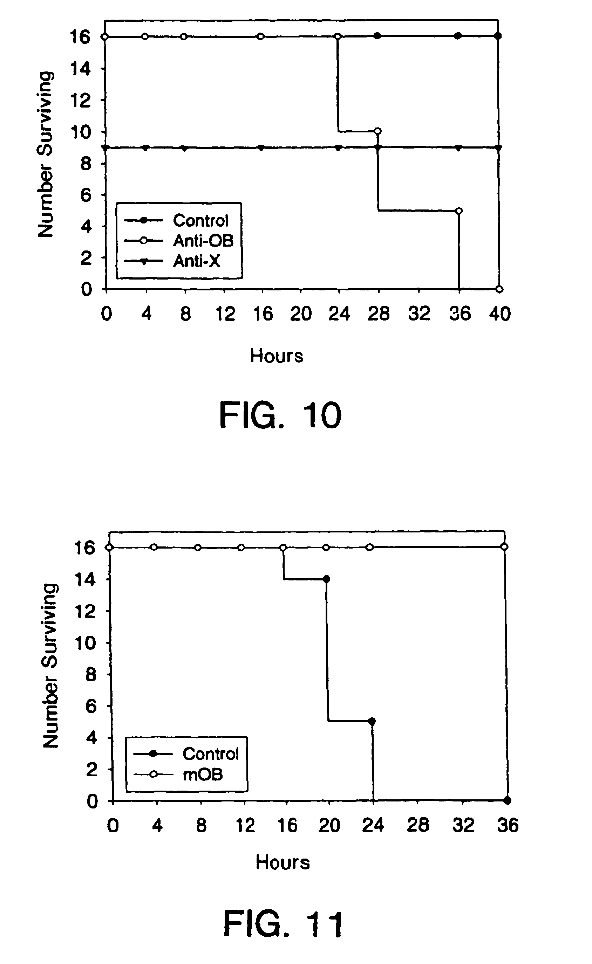

Figure 10 is a graphical representation of the time course of the survival of mice that had received a LPS injection (6 µg per gram of body weight) after pretreatment with either anti-OB protein antiserum (anti-OB, N = 16), preimmune rabbit serum (control, N = 16), or one of three other unrelated rabbit antisera (anti-X, N=9: 3 treated with each antiserum);
Figure 11 is a graphical representation of the time course of the survival of mice that were treated with OB protein (mOB, N = 16) or vehicle (control, N-16) after a LPS injection (10 µg per gram of body weight);

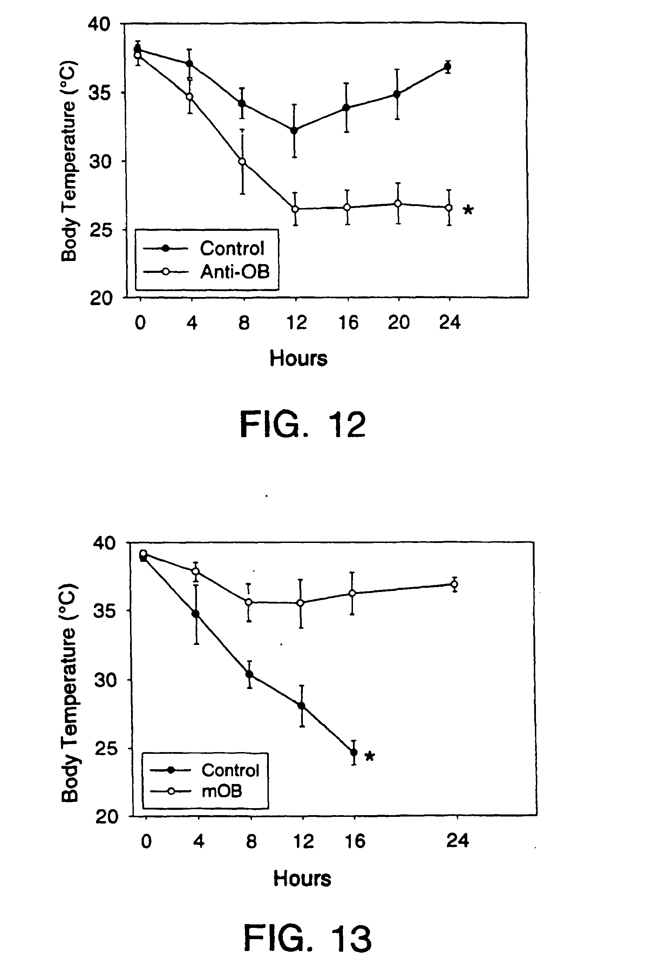

Figure 12 is a graphical representation of the time course of the change in body temperature of mice that had received a LPS injection (6 µg per gram of body weight) after pretreatment with either anti-OB protein antiserum (anti-OB, N=16, except at * where N < 16 due to mortality) or preimmune rabbit serum (control, N=16), data expressed as mean ± S.E.M.;
Figure 13 is a graphical representation of the time course of the change in body temperature of mice that were treated with OB protein (mOB, N = 16) or vehicle (control, N-16, except at * where N < 16 due to mortality) after a LPS injection (10 µg per gram of body weight), data expressed as mean ± S.E.M.;

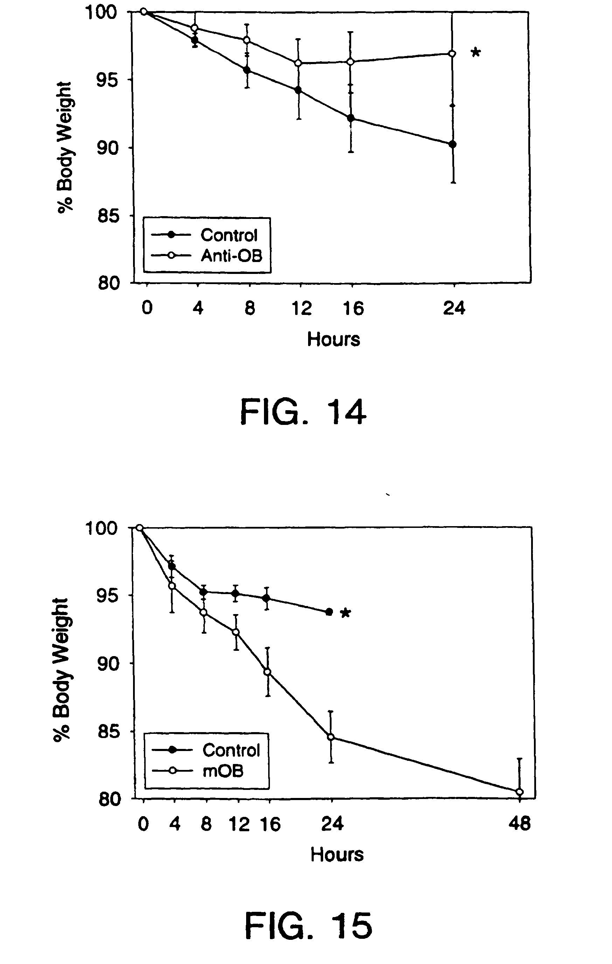

Figure 14 is a graphical representation of the time course of the change in body weight (percent of initial body weight) of mice that had received a LPS injection (6 µg per gram of body weight) after pretreatment with either anti-OB protein antiserum (anti-OB, N = 16) or preimmune rabbit serum (control, N = 16, except at * where N < 16 due to mortality), data expressed as mean ± S.E.M.;
Figure 15 is a graphical representation of the time course of the change in body weight (percent of initial body weight) of mice that were treated with OB protein (mOB, N=16) or vehicle (control, N-16, except at * where N < 16 due to mortality) after a LPS injection (10 µg per gram of body weight), data expressed as mean ± S.E.M.; and

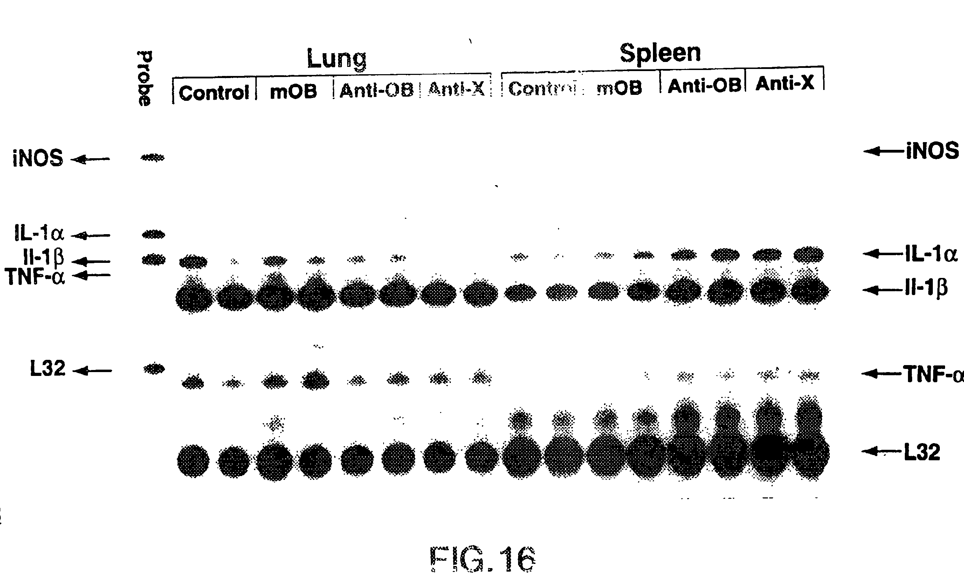

Figure 16 is a representation of an autoradiogram showing the results of a RNase protection assay showing the expression of iNOS, IL-1α, IL-1β, and TNF-α mRNAs in mouse.

### Detailed Description of the Preferred Embodiments

It has been found that substances that initiate or mediate SIRS, for example, LPS and several cytokines, induce the increased expression of OB-R in liver and other peripheral tissues. Thus, occupancy and activation of OB-R by an agonist ligand such as recombinant OB protein, OB-related peptides or peptide conformational analog of human OB protein comprising conservative substitutions of amino acid residues serves as a protective homeostatic mechanism in systemic inflammatory response syndrome conditions such as endotoxic shock, sepsis and septic shock. A preferred OB-R agonist ligand is recombinant OB protein. Suitable therapeutic human doses of recombinant OB protein are from about 1 micrograms per kilogram body weight to about 50 microgram per kilogram body weight. One preferable therapeutic human dose is about 10 micrograms per kilogram body weight.

While the regulation of energy homeostasis is essentially a function of the CNS, food intake and the majority of the energy expenditure take place in peripheral organs such as the liver. It has been found that the OB protein and the OB receptor have a functional involvement in peripheral energy homeostasis. In general, critical illness and trauma can dramatically alter metabolism, with the expression of the OB receptor changing in response to pathological stress. The expression of OB-R in liver and other peripheral organs, but not in the central nervous system, has now been shown to be induced by endotoxic shock produced intravenous injection of cytokines, such as, IL-1β, TNF-α and IL-6, as well as cytokine inducing agents such as LPS, into mice, an accepted animal model of SIRS and related conditions. OB protein, antibodies to OB protein, and OB-R expression inducers are useful for the diagnosis and treatment of conditions such as sepsis, systemic inflammatory response syndrome, cachexia and anorexia.

The administration of recombinant mouse OB protein to mice following OB-R induction with a normally lethal dose of LPS conferred complete resistance to LPS, resulting in survival. The OB-treated mice maintained a higher body temperature and displayed dramatic weight loss in contrast to control counterparts. In vivo administration of OB antisera, on the other hand, elicited the opposite effects by blocking OB-mediated processes, thereby stimulating post-prandial food intake leading to rapid weight gain. Co-administration of LPS with a second in vivo treatment with OB antisera, however, resulted in 100% mortality as compared to animals treated with control antisera.

OB protein, in mediating host responses to LPS-induced endotoxemia, exerts its protective effect primarily by initiating energy mobilization and heat production in critical conditions, the effect of which is proportional to the level of OB protein in the blood. By altering the levels of OB protein, the amount of energy mobilized to resist challenges induced by inflammatory agents is correspondingly altered, thereby effecting the ultimate inflammatory response. -

Therefore, in view of the newly discovered physiological properties of OB protein and OB antibodies in regulating energy mobilization and consumption, the present invention describes both diagnostic and therapeutic uses of recombinant human OB protein and antibodies thereto as described in the claims.

### Diagnostic Applications

OB antibodies are useful for detecting the amount of OB present in sample taken from a patient. The invention provides the use of an antibody capable of binding to OB protein for the manufacture of a composition for detecting a systemic inflammatory response in a patient. One preferred diagnostic embodiment is the use of OB antibodies for detecting the amount of OB present in a blood sample taken from a patient exhibiting a SIRS condition such as sepsis, septic shock, and the like. The determination of OB levels is useful in systemic inflammatory response syndromes (SIRS) that are characterized by an acute increase in inflammatory mediators, such as IL-1β, Il-6, TNF, LPS and the like. Such conditions are noted in preoperative patients subject to fasting, in patients with acute injuries such as burns or trauma, in patients with SIRS, or with ongoing bacterial infections or those receiving TNF-α for treatment of tumors and in persons suffering from hypothermia.

While there is little sequence similarity between OB proteins and other molecules, the three-dimensional conformation of the OB protein molecule is analogous to that of several long-chain helical cytokines: four major alpha helix regions, A-D, connected by short loops and minor helical regions (Zhang, F., et al., Crystal Structure of the obese protein leptin-E100, Nature 387: 206-209 (1997)).

As used herein, a conformational analog of OB protein is a molecule having substantially the same conformational characteristics of its three-dimensional structure that are required for activation of the OB receptor. Examples of such conformational characteristics include the conformation of the A major helix, the conformation of the D major helix, and the disulfide bond that maintains the geometrical relationship between the A and D major helices. Thus, amino acid substitutions that conserve the conformational characteristics of the molecule, for example, in the loop regions connecting the major helices, would produce conformational analogs to OB protein.

Peptides derived from the region of the OB protein from amino acid residues 106 to 140, as short as about 15 amino acids long, have been shown to be effective in mimicking the action of full-length recombinant OB protein (Grasso, P., et al. , In vivo Effects of Leptin-Related Synthetic Peptides on Body Weight and Food Intake in Female ob/ob Mice: Localization of Leptin Activity to Domains Between Amino Acid Residues 106 - 140, Endocrinology 138: 1413-1418 (1997)). As used herein, "OB-related peptides" refers to natural or synthetic peptides derived from the region of the OB protein from about amino acid residue 106 to about amino acid residue 140 and includes conservative amino acid residue substitutions.

The term "antibody" in its various grammatical forms is used herein to refer to immunoglobulin molecules and immunologically active portions of immunoglobulin molecules, i.e., molecules that contain an antibody combining site or paratope. Exemplary antibody molecules are intact immunoglobulin molecules, substantially intact immunoglobulin molecules and those portions of an immunoglobulin molecule that contain the paratope, including those portions known in the art as Fab, Fab' and F(ab')₂ An antibody composition of the present invention is characterized as containing antibody molecules that immunoreact with OB protein or portions thereof.

An antibody composition of the present invention is typically produced by immunizing a mammal with a inoculum of OB protein or some fragment of OB protein, alone or in combination with a suitable adjuvant such as Freund's adjuvant, and thereby induce in the mammal antibody molecules having the appropriate immunospecificity. The antibody molecules are then collected from the mammal and isolated to the extent desired by well known techniques such as, for example, immunoaffinity chromatography. The antibody composition so produced can be used, inter alia, in the diagnostic methods and systems of the present invention or in the preparation of therapeutic compositions of the present invention.

Monoclonal antibody compositions can also be used with the present invention. A monoclonal antibody composition contains, within detectable limits, only one species of antibody combining site capable of effectively binding to OB protein. Thus, a monoclonal antibody composition of the present invention typically displays a single binding affinity for OB protein even though it may contain antibodies capable of binding proteins other than OB protein. Preferred monoclonal antibodies are those that bind to portions of the OB protein that are required for activation of the OB receptor, such as the A helix, the D helix, ,or regions of OB protein that maintain the relative positions of the A and D helices that are required for the activation of the OB receptor. Monoclonal antibodies against human OB protein have been described and their preparation was discussed in (Tsuruo, Y. et. al., Horm. Metab. Res. 28: 753-755 (1996). Monoclonal antibodies are also supplied commercially from vendors on a custom order basis (e.g., Alpha Diagnostic International, Inc., San Antonio, TX). Purified polyclonal anti-OB antibodies are commercially available from several sources (R&D Systems, Minneapolis, MN; Research Diagnostics, Inc., Flanders, N.J.; Linco Research, Inc., St. Charles, MO; Affinity BioReagents, Inc., Golden, CO).

Determination of OB levels with OB antibodies is performed by assay methods, including ELISA, radioimmunoassay (RIA), Western blot analysis, and the like, that are familiar to one of ordinary skill in the art. The determined OB protein levels are then compared to normal levels for the state of the patient, e.g., fasting, time of day, body mass index (BMI), aerobic conditioning, gender, etc. For example, the normal range found for lean males at 8 a.m. was 12.0 ± 4.4 ng/ml (Sinha, M.K., et al., Nocturnal Rise of Leptin in Lean, Obese, and Non-Insulin-dependent Diabetes Mellitus Subjects, J. Clin. Invest. 97: 1344-1347 (1996)). See, also Horn, R. et al., Radioimmunoassay for the detection of leptin in human serum, Exp. Clin. Endocrinol. Diabetes 104: 454-458 (1996); McGregor, G.P., et al., Radioimmunological Measurement of Leptin in Plasma of Obese and Diabetic Human Subjects, Endocrinology 137: 1501-1504 (1996). It has recently been found that OB protein is present in the circulation in both bound and free form, and that the ratio of the two forms is different in lean and obese subjects (Sinha, M.K., et al., Evidence of Free and Bound Leptin in Human Circulation. Studies in Lean and Obese Subjects and During Short-Term Fasting, J. Clin. Invest. 98: 1277-1282 (1996)). The relation of free and bound forms to OB protein biological activity can be considered in the context of OB protein assays.

In an alternative embodiment, immunohistochemical assay of OB-R receptor numbers are performed on tissue biopsy materials using standard protocols. A preferred tissue biopsy is liver biopsy.

### Therapeutic Applications

Applicatons of the present invention, include the use of an OB-R against ligand for the manufacture of a medicament for the treatment of a systemic inflammatory response syndrome (SIRS) condition, wherein the OB-R against ligand is as recited in claim 1. Suitable OB-R agonist ligands include recombinant OB protein, peptide conformational analogs of human OB protein comprising conservative substitutions of amino acid residues and OB-related peptides as recited in claim 1. A preferred OB-R agonist ligand is recombinant human OB protein. A suitable dosage range for recombinant human OB protein is from about 1 microgram per kilogram body weight to about 50 micrograms per kilogram body weight. OB-related peptides are used in a dosage range from about 0.1 microgram per kilogram body weight to about 5 micrograms per kilogram body weight, adjusting the dosage to account for art-recognized differences in potency and solubility (Grasso, P. et al., (1997)).

The mode of administration may be intravenous infusion over an extended time period or a single intravenous or subcutaneous injection. The daily dose may be administered as a single dose or divided into multiple dose given at intervals during the day.

In a further embodiment the invention provides the use of an OB-R agonist ligand in the manufacture of a medicament for reducing the toxic effects of therapeutic cytokines, as recited in claim 5. For example, to counteract the possible toxic side effects of OB-R expression inducers such as therapeutic cytokines, such substances are administered in a composition in combination with OB-R agonist ligands. Administration of such compositions is useful for conditions in with cytokines are normally administered for a therapeutic purpose such as tumor treatment, in order to provide effective protection by the OB-R agonist ligands from undesirable metabolic side effects. The up-regulation of the OB-R allows for the complete therapeutic effect mediated by OB-R agonist ligands such as OB protein. A suitable dosage range for recombinant human OB protein is from about 1 microgram per kilogram body weight to about 50 micrograms per kilogram body weight.

It has been found that OB protein is an important host defense factor against endotoxin stress. The protective effect of OB protein against endotoxin was not caused by suppressing, the expression of major inflammatory mediators, since the mRNA levels of IL-1α, IL-1β, TNF-α, and iNOS in lung and spleen were similar in all LPS-treated mice regardless of the experimental manipulations (Figure 16). A comparison of the four groups of mice revealed striking correlations among the OB protein available , the survival from endotoxin shock (Figures 10 and 11), the maintenance of body temperature (Figures 12 and 13), and the loss of body weight (Figures 14 and 15).

Anti-OB Ab-treated mice showed the least loss of body weight and had the most profound hypothermia even with a relatively low dose of LPS. Conversely, OB-treated mice receiving a high dose of LPS maintained a higher body temperature than those control mice that received less LPS. The OB-treated mice also had a greater weight loss than any reported in the literature (16% in the first 24 hours, compared with an average of 10% reported by other groups). The mice in these tests were age, sex and weight matched, fed the same diet, and, therefore, should have had very similar energy store. The different responses to endotoxemia described were likely due to differences in metabolic energy mobilization and dissipation, which, in turn, were attributed to the experimental manipulation of circulating OB protein levels.

When the level of circulating OB protein is varied, the energy mobilized to resist endotoxin challenge is correspondingly altered, and the outcome of the host response to endotoxin stress is affected. *ob*/*ob* mice, lacking OB protein due to a mutation in the ob gene, were very sensitive to LPS insult: a dose as low as 2 µg per gram of body weight caused a rapid fall of body temperature and death.

The results also suggest the existence of two pathways of thermogenesis and thermostasis. Anti-OB Ab treatment per se did not cause hypothermia in normal mice, suggesting that the thermostasis under non-pathological conditions was largely OB protein-independent. However, when given LPS, the anti-OB Ab-treated mice developed profound hypothermia, indicating that the thermogenesis in response to endotoxemia had become OB protein dependent. A corollary of this model is that genetic defects affecting the OB/OB-R pathway will have a severe hypothermic response to endotoxin. Indeed, *db*/*db* mice which carry a mutation in the OB-R gene, responded to a low dose of LPS injection in a manner very similar to that seen in *ob*/*ob* mice (data not shown), despite their increased level of OB expression.

### Example 1: Induction of OB-R Expression by Administration of LPS

The injection of LPS and cytokines, substances that are associated with sepsis, septic shock or SIRS caused increases in the expression of the OB receptor in peripheral organs such as liver, but not in brain.

Intravenous injection of lipopolysaccharide (LPS), IL-1β, TNF-α and IL-6 to mice induced OB receptor expression in the liver and other peripheral organs, but not in the central nervous system (CNS). To investigate the functional significance of the increased OB-R expression, an anti-OB antiserum was used to neutralize endogenous OB protein in mice prior to an LPS injection. The neutralization of OB protein led to profound hypothermia, insignificant loss of body weight, and death in mice in response to an otherwise nonlethal dose of LPS. Conversely, mice administered recombinant mouse OB protein became more resistant to LPS and survived an otherwise lethal dose. The OB protein-treated mice maintained a relatively high body temperature and displayed a dramatic weight loss. These results suggest that OB protein may promote energy mobilization to compensate for the increased energy consumption in endotoxemia, and that the OB/OB-R pathway may play an important role in critical host responses to inflammatory stress.

### Methods:

In general, standard techniques or published modifications were used; see, generally, Sambrook, J., et al., Molecular Cloning A Laboratory Manual, 2d Ed., Cold Spring Harbor Laboratory Press (1989). C57BL/6 mice, 5-8 weeks of age and 17-20 g of weight, were injected intravenously with either LPS (5 µg per gram of body weight, List Biological Laboratory, Campbell, CA), IL-1β (R&D Systems, Minneapolis, MN), IL-6 (2.5 µg, Pharmingen, San Diego, CA), or TNF-α (a gift from Genentech, San Francisco, CA). Animals were sacrificed at 0, 4, 8 or 24 hours, or 2, 3 or 5 days after the injection.

Tissue from various organs, including brain, liver and kidney, was dissected and snap-frozen in liquid nitrogen. Total RNA was prepared from the frozen tissues by a single-step method (Chomczynski, P. & Sacchi, N., Single-Step Method of RNA Isolation by Acid Guanidinium Thiocyanate-Phenol-Chloroform Extraction, Analytical Biochemistry 162: 156-159 (1987).

The RNase protection assays were carried out as previously described (Feng, L., et al., Alternative Splicing of the NC1 Domain of the Human α3(IV) Collagen Gene J. Biol. Chem. 269: 2342-2348 (1994); Xia, Y., et al., LPS-Induced MCP-1, IL-1β, and TNF-α mRNA Expression in Isolated Erythrocyte-Perfused Rat Kidney, Am. J. Physiol. 264: F774-F780 (1993)). A. ten microgram aliquot of total RNA pooled from three similarly treated mice was used for each sample in the RNase protection assay. The stored pooled samples were dissolved in 10 µl of 80% formamide, 0.4 M NaCl, 1 mM EDTA, and 40 mM piperazine-N,N'-bis(2-ethanesulfonic acid), heated to 85 degrees Celsius for 5 minutes. Each ten microgram sample was then hybridized with about 1 x 10⁵ cpm (counts per minute) of the appropriate [³²P]UTP-labeled antisense riboprobe at 55 degrees Celsius for at least 10 hours. The unhybridized RNA was then digested with 50 unit/ml RNase T1 (GIBCO/BRL, Gaithersburg, MD) and 24 µg/ml RNase A at 30 degrees Celsius for one hour. The RNase was then digested with 625 µg/ml proteinase K (Boehringer Mannheim, Indianapolis, IN) for 30 minutes at 37 degrees Celsius. After phenol-chloroform extraction and sodium-acetate-ethanol precipitation, the protected hybridized RNA was denatured and electrophoresed on a 10% polyacrylamide gel. The gels were transferred to 3M Whatman filter paper, dried and exposed to Kodak X-Omat film. The resulting autoradiograms were developed in a Kodak X-Omat processor were used only for qualitative screening.

Radioactivity due to hybridization of target sequences with ³²P-labeled riboprobes was quantified by scanning the gels on an AMBIS radioanalytic scanning system (AMBIS Systems, San Diego, CA).

An OB-Rb cDNA probe (from base 2548 to base 2835 of OB-Rb, Gen-Bank™ Accession No. U46135) was subcloned from a full-length mouse OB-Rb cDNA. The full-length mouse cDNA for the OB-R long form (OB-Rb) was cloned from a mouse hypothalamus cDNA library (Stratagene, La Jolla, CA), and the sequence was verified against that of U46135.

The full-length mouse cDNA for the OB-R short form (OB-Ra) was cloned from a mouse lung cDNA Library (Stratagene, La Jolla, CA). A 224 bp probe that included base 1250 to base 1474 (as indicated on the OB-Rb sequence) of OB-Ra was used for the RNase protection assay. This fragment, which comprises a sequence that is shared by all variants of OB-R, was used as a probe for the total level of OB-R (OBR₍ₜ₎).

The expression of other forms of OB-R mRNA was analyzed using selective probes for the respective different forms of OB-R. The designated probes provided full protection to their corresponding OB-R forms and partial protection for other OB-R forms. A probe derived from L32 (33-126, Gen-Bank™ Accession No. XO6483), a housekeeping gene encoding ribosomal protein, was used as a control.

OB-Rc and OB-Re probes were cloned by reverse-transcription-PCR (RT-PCR) of total liver RNA from LPS-treated C57BL/6 mice. Protocols for RT-PCR are known in the art (for example, pages 15-13 - 15-15 of Ausubel, F.M., et al., Short Protocols in Molecular Biology. 2nd Edition, John Wiley and Sons, New York, (1992)).

One suitable protocol for RT-PCR is a modification of that previously described (Feng, L., et al., J. Biol. Chem. 269: 2342-2348, 1994). The primers used in RT-PCR are listed in Table 1, below. Primer oligonucleotides were synthesized using an ABI model 380B synthesizer (Applied BioSystems, Foster City, Ca).

**Table 1: PCR Primers**

| | **Sequence** | |
|---|---|---|
| **OB-Rc sense** | 5'-GCTATCGACAAGCAGCAGAAT-3' | (SEQ ID NO. 8) |
| **OB-Rc antisense** | 5'-TGAACACAACAACATAAAGCCC-3' | (SEQ ID NO. 9) |
| **OB-Re sense** | 5'-TGTTATATCTGGTTATTATTGAATGG-3' | (SEQ ID NO. 10) |
| **OB-Re antisense** | 5'-CATTAAATGATTTATTATCAGAATTGC-3' | (SEQ ID NO. 11) |

First strand cDNA synthesis was performed using total liver RNA from LPS-treated C57BL/6 mice and murine leukemia virus reverse transcriptase with a random hexanucleotide primer. The 100 µl reaction mixture contained standard enzyme buffer, 5 µg of total RNA, 20 units of RNasin (RNase inhibitor), 500 pmol of hexanucleotide primer, 10 mM dithiothreitol, 1 mM of each dNTP, with 200 units of reverse transcriptase. Each reaction mixture was heated to 95 degrees Celsius for 10 minutes. PCR was then performed with separate aliquots of the reaction mixture with the appropriate primers for 35 cycles, using 60 degrees Celsius for annealing.

The cDNA segments used to generate riboprobes were excised by the appropriate restriction endonucleases and subcloned into the multiple cloning site of a standard transcription vector. Suitable transcription vectors include a vector chosen from the pGEM series (Promega, Madison, WI). Labelled single stranded riboprobes were synthesized using standard in vitro transcription protocols, either those provided by the manufacturer or other standard protocols (e.g. Ausubel, F.M., et al., pages 4-18 - 4-21) with the appropriate (e.g., SP6 or T7) bacteriophage RNA polymerase. The riboprobes contained regions corresponding to the vector polylinker in addition to the region corresponding to the target sequence, and thus were longer than the protected bands. The mouse ribosomal L32 gene, a constitutively expressed "housekeeping" gene, was used throughout the study as a control.

### Results:

When LPS was administered to C57BL/6 mice, a strong induction of total OB-R expression (OB-R₍ₜ₎) was detected in a number of peripheral organs (Figure 1), but not in several areas of the central nervous system, such as the hypothalamus, that are known to express OB-R (compare Figure 1 and Figure 6). The increased expression of OB-R was most prominent in the liver, the major site of metabolic regulation. The increase of OB-R mRNA expression in the liver was LPS dose-dependent, and peaked between 24 and 48 hours post-LPS injection (Figure 1).

Unexpectedly, RNase protection assays using probes specific for alternatively spliced forms of OB-R mRNA revealed that the long form, OB-Rb, was also induced in the liver to a level comparable to that found in the *ob*/*ob* mouse hypothalamus and greater than that of the lean control mouse hypothalamus (Figure 2). However, the majority of hepatic OB-R were the OB-Ra, OB-Rc (Figure 3) and OB-Re (Figure 4) forms. OB-Rd expression in the liver was undetectable (data not shown).

In addition to LPS, OB-R expression was induced by the cytokines IL-6, IL-1α and TNF-α (Figure 5). Contrary to a recent report (12), we found no detectable increase in OB expression in white adipose tissue in LPS-treated mice, but detected a distinct induction of OB mRNA expression in the adrenal gland (Figure 4). No OB mRNA expression was found in the brain, heart, lung, liver, kidney, spleen, muscle, stomach, duodenum, jejunum, ileum, or colon of LPS-treated mice (data not shown).

### Example 2: Production of Recombinant OB Protein

Recombinant OB protein was expressed in E. coli using a prokaryotic expression vector and extracted from inclusion bodies. Other vectors and host cells systems, including eukaryotic cells, are known in the art and also suitable for the expression of OB protein. See, generally, Ausubel, F.M., et al. Short Protocols in Molecular Biology, 2nd Ed., pages 16-1 to 16-89.

The coding region of mouse OB cDNA (65-619, Gen-Bank™ Accession No.U18812) was cloned by RT-PCR of total RNA from C57BL/6 white adipose tissue. The coding region was subcloned in expression vector, pETM1 (Feng, L., et al., J. Biol. Chem. 269: 2342-2348, 1994), to express a His-tagged recombinant mouse OB protein. The construction of pETM1 from the commercially available vector pET-11a (Novagen, Madison, WI) is illustrated on Figure 8.

After the expression of OB protein is induced, the bacteria were harvested and the inclusion bodies were extracted with a buffer containing 6M urea. The extract was loaded on a Ni-NTA affinity column (Qiagen, Chatsworth, CA) and the purification procedure was carried out as previously described (Feng, et al.(1994)). The protein was refolded on the column by adding refolding buffer containing 5 mM CaCl₂/20 mM Tris/0.2 NaCl with an urea gradient of 4 M - 0.5 M at a rate of about 0.5 ml/minute. After refolding, the protein was eluted with 80 mM imidazole/5 mM CaCl₂/20 mM Tris/0.2 NaCl/ 0.5 mM urea and then dialyzed against phosphate-buffered saline (PBS). Polyclonal antibodies was raised by immunizing a rabbit with the recombinant mouse OB and Freund's adjuvant using standard procedures. Antiserum was used in the following examples.

### Example 3: Effects of Anti-OB Antibodies on Metabolism

Intravenous administration of antibodies directed against OB protein effectively opposes the effects of endogenous OB protein.

Recombinant mouse OB protein was produced in an E. coli expression system as described in Example 2, and was used to generate rabbit polyclonal anti-OB antibody. The antibody, when injected intravenously into mice, stimulated food intake, leading to rapid weight gain, and thus was effective in blocking OB protein function. The results are shown in Figure 9.

Female C57BL/6 mice, 6-8 weeks of age and 15-17 g of weight, were group housed four per cage and adapted to a 12:12 hour light: dark cycle (light from 6:00 to 18:00). Mice were given a daily intravenous injection of 0.2 ml anti-OB antiserum or preimmune rabbit sera ("vehicle") at 10 p.m., after their initial dark phase food intake. Their body weight was measured at 10 a.m. the next day.

The weight gain induced by anti-OB antiserum is illustrated in Figure 9. Data are expressed as mean ± S.E.M. (N=8). While the weight of the control group remained essentially constant over the week, the anti-OB treated group showed a weight increase at the first weighing, which continued for the entire study period.

### Example 4: Effects of Anti-OB Antibodies and Recombinant OB-Protein on Response to Endotoxic Shock

The fact that the OB-R variants induced in the liver were predominantly short forms raised the question of functional relevance of the hepatic OB-R expression. OB-Rb is the main form expressed in the hypothalamus, while the choroid plexus expresses only OB-Ra. That the mutation in *db*/*db* mice affects OB-Rb, but not OB-Ra, suggests that OB-Rb is crucial for regulating food intake and OB-Ra may act as an OB protein transporter. Accordingly, the prominent expression of OB-R in the liver could initiate intracellular signal transduction or, alternatively, mediate the clearance of OB protein. We found that administration of neutralizing anti-OB antibody (Ab) or OB protein to LPS-treated mice distinguished between the two alternatives.

Male C57BL/6 mice, 5-8 weeks of age and 17-21 g of body weight, were used for this study. For antisera treatment, mice were given an i.v. injection of 200 µl rabbit antisera. LPS at a dose of 6 µg per gram of body weight was then co-injected with a second dose of anti-OB antibody to the pretreated mice. LPS was dissolved in antisera at a concentration of 0.6 mg/ml and was injected intravenously 4 hours after the initial antisera treatment. Food was retrieved from mouse cages during the 4-hour pretreatment period to prevent any food intake differences resulting from anti-OB antiserum-induced hyperphagia, and was added back after LPS injection.

While this dose of LPS was not lethal to C57BL/6 mice treated with preimmune rabbit serum, all the mice in the anti-OB Ab-treated group died within 40 hours. Figure 10 illustrates the results from three groups of mice: those pretreated with anti-OB antiserum (anti-OB, N=16), preimmune rabbit sera control, N=16), or three other unrelated rabbit antisera ("Anti-X", N=3 for each antiserum). This LPS sensitizing effect was specific for anti-OB Ab, since mice treated similarly with three other unrelated antibodies ("Anti-X") all survived (Figure 10).

In comparison, mice treated with OB protein (5 µg per gram of body weight mOB, N = 16) were able to survive a higher dose of LPS (10 µg per gram of body weight) that was fatal to the control group of mice receiving the vehicle alone (control, N=16). Mouse OB protein and LPS were prepared in saline at a concentration of 0.5 mg/ml and 1 mg/ml, respectively, and injected intravenously into the mice. A vehicle solution used for OB protein dialysis was injected into control mice. To eliminate any circadian effect, experiments at different days were all started at the same hours. Mice were examined at 4-hour intervals post LPS injection for the first 24 hours, and survival was monitored for 7 days following the LPS injection. The results are shown in Figure 11. The dose of LPS killed all the mice in the control group within 24 hours. However, in the experimental group, OB protein treatment conferred mice complete resistance to this dose of endotoxin. The OB-treated mice displayed noticeably less severe symptoms of endotoxemia, remaining alert and responsive to touch and other manipulation, and recovering quickly.

The effects of both OB protein and anti-OB antibodies on body temperature and body weight were monitored in the same groups of mice. Core body temperature measurements were made by insertion of a thermistor probe (Yellow Springs Instrument, Yellow Springs, Ohio) into the colon, 1.5 cm beyond the rectum. Body weight measurements were made on a portable digital balance (Ohaus, Florham Park, NJ).

The mice receiving anti-OB antibodies, which ultimately died, showed lower body temperature (Figure 12) and less weight loss (Figure 14) than the corresponding control group than survived. Conversely, the mice receiving OB-protein, and which survived the endotoxic shock, showed higher body temperature (Figure 13) and more weight loss (Figure 15) than the corresponding control group which succumbed.

While administration of both OB protein and anti-OB antibodies had significant effects on survival, body weight and body temperature, there was little effect on the expression of iNOS, IL-1α, IL-1β, and TNF-α mRNAs in lung and spleen (Figure 16). Mice were treated as described in above (Figures 10-15).

RNase protection assays were performed as described in Example 1. Each sample was 5 µg total RNA was used for each sample in the RNase protection assay. Riboprobes were produced as described in Example 1 based on the following cDNA fragments: IL-1α (from base 172 to base 366, Accession No. X01450, SEQ ID NO. 3), IL-1β (from base 500 to base 671, Accession No. M15131, SEQ ID NO. 4), TNF-α (from base 428 to base 557, Accession No. M11731, SEQ ID NO. 5), mouse iNOS (from base 2404 to base 2698, Accession No. M92649, SEQ ID NO. 6), and L32 (from base 33 to base 126, Accession No. X064383, SEQ ID NO. 7).

Relatively little change in the pattern of expression of these markers was observed (Figure 16), suggesting that these the protective actions of OB proteins are direct and not indirect and mediated by these cytokines.

The foregoing is intended to be illustrative of the present invention, but not limiting. Numerous variations and modifications of the present invention may be effected without departing from the scope of the invention, as defined by the claims.

## Claims

1. The use of an OB-R agonist ligand for the manufacture of a medicament for the treatment of a systemic inflammatory response syndrome (SIRS) condition, wherein said OB-R agonist ligand is selected from:
recombinant human OB protein;
a peptide conformational analog of human OB protein comprising conservative substitutions of amino acid residues; or
a peptide derived from the region of OB protein from amino acid residue 106 to amino acid residue 140, optionally including conservative amino acid substitutions.

2. The use of claim 1 wherein the OB-R agonist ligand is recombinant human OB protein for administration in an amount from 1 microgram per kilogram body weight to 50 micrograms per kilogram body weight.

3. The use of claim 1 wherein the OB-R agonist ligand is a peptide derived from the region of OB protein from amino acid residue 106 to amino acid residue 140, optionally including conservative amino acid substitutions.

4. The use of claim 1 wherein the SIRS condition is sepsis.

5. The use of an OB-R agonist ligand in the manufacture of a medicament for reducing the toxic effects of therapeutic cytokines, wherein said OB-R agonist ligand is selected from:
recombinant human OB protein;
a peptide conformational analog of human OB protein comprising conservative substitutions of amino acid residues; or
a peptide derived from the region of OB protein from amino acid residue 106 to amino acid residue 140, optionally including conservative amino acid substitutions.

6. The use of claim 5 wherein the OB-R agonist ligand is recombinant human OB protein.

7. The use of claim 6 wherein the amount of recombinant human OB protein per dose is 1 microgram per kilogram body weight to 50 micrograms per kilogram body weight.

8. The use of an antibody capable of binding to OB protein for the manufacture of a composition for detecting a systemic inflammatory response in a patient.

## Patentansprüche

1. Verwendung eines agonistischen Liganden von OB-R zur Herstellung eines Medikaments zur Behandlung eines Zustands eines systemischen inflammatorischen Response-Syndroms (SIRS), wobei besagter agonistischer Ligand von OB-R ausgewählt ist aus:
rekombinantem OB-Protein vom Menschen,
einem zum OB-Protein vom Menschen konformationsanalogen Peptid, das konservative Substitutionen von Aminosäuren umfasst oder
einem Peptid, das von der Region des OB-Proteins von Aminosäurerest 106 bis Aminosäurerest 140 abgeleitet ist und wahlweise konservative Aminosäuresubstitutionen enthält.

2. Verwendung nach Anspruch 1, wobei der agonistische Ligand von OB-R rekombinantes OB-Protein vom Menschen zur Verabreichung in einer Menge von 1 Mikrogramm pro Kilogramm Körpergewicht bis 50 Mikrogramm pro Kilogramm Körpergewicht ist.

3. Verwendung nach Anspruch 1, wobei der agonistische Ligand von OB-R ein Peptid, das von der Region des OB-Proteins von Aminosäurerest 106 bis Aminosäurerest 140 abgeleitet ist und wahlweise konservative Aminosäuresubstitutionen enthält, ist.

4. Verwendung nach Anspruch 1, wobei es sich bei dem SIRS-Zustand um Sepsis handelt.

5. Verwendung eines agonistischen Liganden von OB-R zur Herstellung eines Medikaments zum Verringern der toxischen Wirkungen therapeutischer Zytokine, wobei besagter agonistischer Ligand von OB-R ausgewählt ist aus:
rekombinantem OB-Protein vom Menschen,
einem zum OB-Protein vom Menschen konformationsanalogen Peptid, das konservative Substitutionen von Aminosäuren umfasst oder
einem Peptid, das von der Region des OB-Proteins von Aminosäurerest 106 bis Aminosäurerest 140 abgeleitet ist und wahlweise konservative Aminosäuresubstitutionen enthält.

6. Verwendung nach Anspruch 5, wobei der agonistische Ligand von OB-R rekombinantes OB-Protein vom Menschen ist.

7. Verwendung nach Anspruch 6, wobei die Menge von rekombinantem OB-Protein vom Menschen pro Dosis 1 Mikrogramm pro Kilogramm Körpergewicht bis 50 Mikrogramm pro Kilogramm Körpergewicht beträgt.

8. Verwendung eines Antikörpers, der OB-Protein binden kann, zur Herstellung einer Zusammensetzung zum Feststellen einer systemischen inflammatorischen Reaktion bei einem Patienten.

## Revendications

1. Utilisation d'un ligand d'un agoniste de OB-R pour la fabrication d'un médicament pour le traitement d'une condition de syndrome de réponse inflammatoire généralisée (SIRS), où ledit ligand d'un agoniste de OB-R est sélectionné parmi :
une protéine OB humaine recombinante
un peptide analogue conformationnel de la protéine OB humaine comprenant des substitutions conservatives de résidus d'acides aminés ; ou
un peptide dérivé de la région de la protéine OB à partir du résidu d'acide aminé 106 jusqu'au résidu d'acide aminé 140, comprenant facultativement des substitutions conservatives d'acides aminés.

2. Utilisation de la revendication 1 où le ligand de l'agoniste de OB-R est la protéine OB humaine recombinante pour administration en une quantité de 1 microgramme par kilogramme de poids corporel à 50 microgrammes par kilogramme de poids corporel.

3. Utilisation de la revendication 1 où le ligand de l'agoniste de OB-R est un peptide dérivé de la région de la protéine OB du résidu d'acide aminé 106 au résidu d'acide aminé 140, comprenant facultativement des substitutions conservatives d'acides aminés.

4. Utilisation de la revendication 1 où la condition de SIRS est une septicémie.

5. Utilisation d'un ligand d'un agoniste de OB-R dans la fabrication d'un médicament pour réduire les effets toxiques des cytokines thérapeutiques, où ledit ligand d'un agoniste de OB-R est sélectionné parmi :
protéine OB humaine recombinante ;
un peptide analogue conformationnel de la protéine OB humaine comprenant des substitutions conservatives de résidus d'acides aminés ; ou
un peptide dérivé de la région de la protéine OB du résidu d'acide aminé 106 au résidu d'acide aminé 140, comprenant facultativement des substitutions conservatives d'acides aminés.

6. Utilisation de la revendication 5 où le ligand de l'agoniste de OB-R est la protéine OB humaine recombinante.

7. Utilisation de la revendication 6 où la quantité de la protéine OB humaine recombinante par dose est de 1 microgramme par kilogramme de poids corporel à environ 50 microgrammes par kilogramme de poids corporel.

8. Utilisation d'un anticorps capable de se lier à la protéine OB pour la fabrication d'une composition pour la détection d'une réponse inflammatoire généralisée chez un patient.
